(19) Europäisches Patentamt
European Patent Office
Office européen des brevets

(11) EP 4 707 344 A2

(12) # EUROPEAN PATENT APPLICATION

(43) Date of publication:
**11.03.2026 Bulletin 2026/11**

(21) Application number: **25203508.4**

(22) Date of filing: **09.08.2024**

(51) International Patent Classification (IPC):
***C09D 5/14*** *(2006.01)*

(52) Cooperative Patent Classification (CPC):
(C-Sets available)
**A01N 43/90; A01N 47/44; A01P 1/00; A01P 3/00; C09D 5/14; C09D 7/63;** C08K 5/3472 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.08.2023 EP 23191982**
**26.10.2023 EP 23206267**

(62) Document number(s) of the earlier application(s) in accordance with Art. 76 EPC:
**24754695.5 / 4 701 418**

(71) Applicant: **CM4Cure s.a.**
**4000 Liège (BE)**

(72) Inventors:
- **OURY, Cécile**
**Liège (BE)**
- **LANCELLOTTI, Patrizio**
**Liège (BE)**
- **AQIL, Abdelhafid**
**Liège (BE)**
- **DITKOWSK, Bartosz**
**Liège (BE)**

(74) Representative: **Barker Brettell LLP**
**100 Hagley Road**
**Edgbaston**
**Birmingham B16 8QQ (GB)**

(54) **NEW HIGH-EFFICIENCY ANTIMICROBIAL COMPOSITION**

(57) New high-efficiency synergic or synergistic antimicrobial composition and application thereof.

Medical device, biomaterial implants or bioprosthesis comprising the new high-efficiency antimicrobial composition incorporated in a coating or bulk distributed.

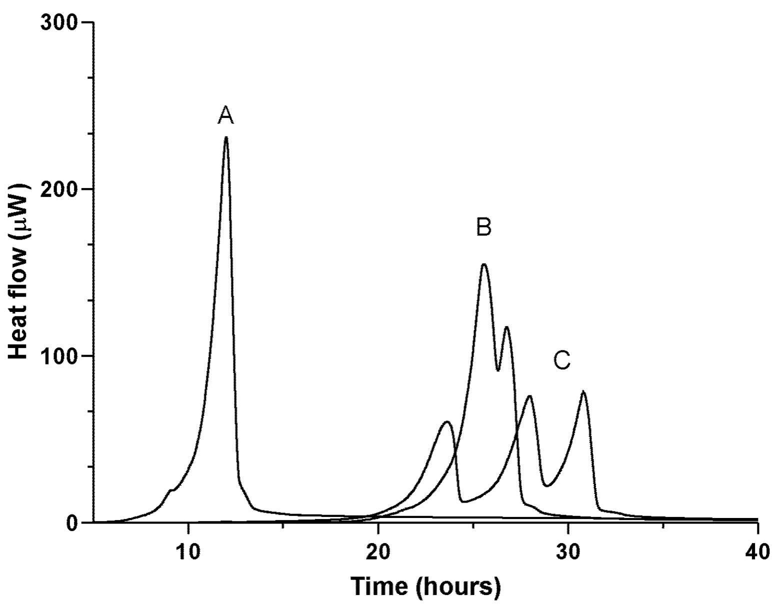


Fig.6



Processed by Luminess, 75001 PARIS (FR)

(Cont. next page)

(52) Cooperative Patent Classification (CPC): (Cont.)

C-Sets
**A01N 43/90, A01N 47/44**

## Description

FIELD OF THE INVENTION

The present invention relates to a new high-efficiency synergic or synergistic antimicrobial composition and application thereof, particularly for treatment or prevention of infection or inflammation on human or animal, more particularly on human or animal skin.

The present invention also relates to a medical device, biomaterial implants or bioprosthesis comprising the new high-efficiency antimicrobial composition incorporated in a coating or bulk distributed.

The present invention also relates to a method of microbial killing or prevention of microbial growth on a surface, particularly on medical device, more particularly on catheter.

BACKGROUND OF INVENTION

Antimicrobial composition is used against pathogenic microorganisms including bacteria, viruses or fungi or a mixture thereof, for prevention or treatment of various infection and disease to host mammal (human and animal).

But microorganisms are becoming more and more resistant to antimicrobial composition used either as antibacterial or antifungal agent.

For example, bacteria are becoming resistant to antibacterial agent belonging to Penicillins, Methicillins, Carbapenems, Cephalosporins, Quinolones, Aminoglycosides, and Glycopeptides, and an increasing number of infections are becoming difficult to cure.

Particularly, coagulase-negative staphylococci (CoNS) are the most frequent bacteria of the normal flora of the skin. These bacteria are common contaminants in clinical specimens and are recognized as agents of clinically significant infection, including bacteremia and endocarditis. Patients at particular risk for CoNS infection include those with prosthetic devices, pacemakers, intravascular catheters, and immunocompromised hosts.

Coagulase-negative staphylococci account for approximately one-third of bloodstream isolates in intensive care units, making these organisms the most common cause of nosocomial bloodstream infection.

Enterococcal species can cause a variety of infections, including urinary tract infections, bacteremia, endocarditis, and meningitis. Enterococci are relatively resistant to the killing effects of cell wall-active agents (penicillin, ampicillin, and vancomycin) and are impermeable to aminoglycosides.

Vancomycin-resistant enterococci (VRE) are an increasingly common and difficult-to-treat cause of hospital-acquired infection.

Multiple epidemics of VRE infection have been described in diverse hospital settings (*e.g.*, medical and surgical intensive care units, and medical and pediatric wards) and, like methicillin-resistant *Staphylococcus aureus,* VRE is endemic in many large hospitals.

Besides human medicine, companion animals, such as cats, dogs, and horses, can also be colonized and infected by microorganism such as MRSA (methicillin resistant), without host adaptation, and therefore may act as reservoirs for human infections. Bacteria can also develop distinct resistance when hosted by animals.

Fungal pathogen such as *C. albicans, Aspergillus fumigatus* and *Cryptococcus neoformans* are also becoming resistant to antifungal agent .

Particularly *C. albicans* is inherently resistant to the majority of known antifungal drugs comprising various azole derivatives (fluconazole, isavuconazile, itraconazole, Posaconazole, voriconazole), but also to polyenes such as amphotericin B and even chlorhexidine (CHX) belonging to the Bisbiguanide antifungal agents having both antifungal and antibacterial activity.

Studies have reported CHX resistance also in different bacterial species, when used at low concentrations

Moreover, chlorhexidine is insoluble in water and need to be formulated with either gluconic or acetic acid to form water soluble digluconate or diacetate salts. Chlorhexidine also requires high concentration to be efficient and may cause skin irritation or allergic reaction in human or animal.

Chlorhexidine may also not be effective against some microorganisms such as gram-negative bacteria or fungi and may not kill microorganism quickly as reported in the following table disclosed by TM KARPINSKI and AK SZKARADKIEWICZ in European Review for Medical and Pharmacological Sciences 2015; 19:1321-1326.

table 1: Minimal Inhibitory Concentration (MIC) of chlorhexidine against various microorganisms.

| Microorganism | MIC of chlorhexdine ***(μg/mL)*** |
|---|---|
| *Staphylococcus aureus* MSSA | 0.25-8 |
| *Staphylococcus aureus* MRSA | 2-8 |
| *Enterococcus faecalis* | 4-16 |

(continued)

| Microorganism | MIC of chlorhexdine ***(μg/mL)*** |
|---|---|
| *Streptococcus mutans* | 0.9-4 |
| *Lactobacillus reuteri* | 0.125-4 |
| *Lactobacillus fermentum* | 0.25-1 |
| *Lactobacillus acidophilus* | 0.5-2 |
| *Porphyromonas gingivalis* | 0.9 |
| *Fusobacterium nucleatum* | 1.8 |
| *Escherichia coli* | 2-16 |
| *Klebsiella* spp. | 8-16 |
| *Pseudomonas aeruginosa* | 16-32 |
| *Candida albicans* | 1-16 |
| *Candida tropicalis* | 75 |
| *Candida krusei* | 150 |
| *Aspergillus* spp. | 8-64 |

Medical device treated with chlorhexidine can result in allergic reactions, including life-threatening anaphylaxis as reported in EP2968677B1.

Despite such drawbacks, CHX has been and continues to be used broadly for disinfecting surfaces in medical devices as well as directly on skin of humans and animals, but there is a need for high efficiency antimicrobial composition having a broad spectrum of action and lower toxicity to the human or animal.

High efficiency antimicrobial composition is necessary to wound care to prevent or treat infection at the wound and promote wound healing.

Antimicrobial composition may be applied on human or animal skin for a large number of reasons including surgery, injury, burns, ulcers, mucosa and the like.

Antimicrobial composition may also be applied on the surface of medical device biomaterial implants or bioprosthesis to prevent infection during insertion or after implant in the human or animal body.

The prevention approach may involve coating of the medical devices, biomaterial implants or bioprosthesis with an antimicrobial coating that includes sufficient antimicrobial agent to maintain sufficient antimicrobial effect for the duration of the implanted or inserted device within the human or animal body.

In 2017, C. Oury and P.Lancellotti describe in EP3509598B1 a new use of triazolo(4,5-D)pyrimidine derivatives for prevention and treatment of bacterial infection, but triazolo(4,5-D)pyrimidine derivatives such as Triafluocyl or Fluometacyl are generally not soluble in an aqueous environment or aqueous medium and requires high concentration to get a high efficiency antimicrobial or bactericidal effect.

The increasing resistance of microorganisms to antimicrobial agent has increased the demand for new high efficiency antimicrobial agent exhibiting both antimicrobial efficacy and anti-antimicrobial resistance. As this demand is not easily met by a conventional one-target-one molecule approach, other approaches are required as the multi-target antimicrobials.

## SUMMARY OF INVENTION

It has been surprisingly found that Triazolo(4,5-d)pyrimidine derivative combined with a bisbiguanide, particularly chlorhexidine and alexidine, provides a synergic or synergistic antimicrobial activity already at low concentration.

The object of the present invention is to provide a high-efficiency synergic or synergistic antimicrobial composition and its application, particularly its use in prevention or treatment of microbial infection or inflammation on human or animal, more particularly on human or animal skin. The synergic or synergistic antimicrobial composition has a broad spectrum of action at low concentration, with reduced allergic reactions and is efficient for killing microbial germs, particularly *Candida albicans, Staphylococcus aureus, Pseudomonas aeruginosa* on surface of objects and human or animal skin. The synergic or synergistic antimicrobial composition is advantageously with a lower risk of antimicrobial resistance.

The object of the invention is also to provide a medical device, biomaterial implants or bioprosthesis comprising the synergic or synergistic antimicrobial composition and method of coating thereof. The method of coating includes applying the antimicrobial composition to at least a portion of the medical device, biomaterial implants or bioprosthesis either in a coating or bulk distributed.

The object of the invention is also to provide a medical device, biomaterial implant or bioprosthesis with antimicrobial properties for use in prevention or treatment of microbial infection or inflammation on human or animal,

particularly on human or animal skin.

Finally, the present invention also refers to a coating of medical device, biomaterial implants or bioprosthesis comprising the antimicrobial composition and to a wound dressing comprising the antimicrobial composition and its application for use in prevention or treatment of microbial infection or inflammation on human or animal.

DETAILLED DESCRIPTION

**According to a first aspect** of the invention, there is provided an antimicrobial composition comprising a synergic or synergistic combination of:

Triazolo(4,5-d)pyrimidine derivative of formula (I)

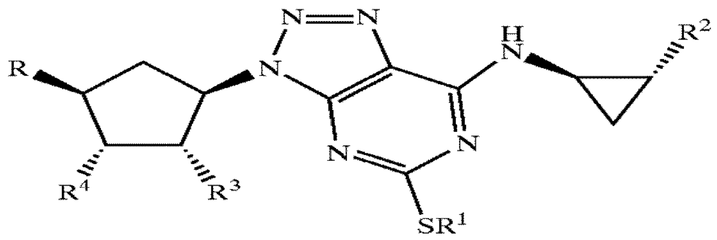

(I)

wherein $R_1$ is C $_{3-5}$ alkyl optionally substituted by one or more halogen atoms; $R_2$ is a phenyl group, optionally substituted by one or more halogen atoms; $R_3$ and $R_4$ are both hydroxyl; R is XOH, wherein X is $CH_2$, $OCH_2CH_2$, or a bond;

or a pharmaceutical acceptable salt or solvate thereof, or a solvate of such a salt provided that when X is $CH_2$ or a bond, $R_1$ is not propyl; when X is $CH_2$ and $R_1$ $CH_2CH_2CF_3$, butyl or pentyl, the phenyl group at $R_2$ must be substituted by fluorine; when X is $OCH_2CH_2$ and $R_1$ is propyl, the phenyl group at $R_2$ must be substituted by fluorine;

together with a biguanide, preferably a bisbiguanide selected from the group consisting of chlorhexidine, alexidine or polyhexylbiguanidine;

for use in prevention or treatment of infection or inflammation on human or animal, particularly on human or animal skin.

The term derivative as used herein refers to a similar structurally Triazolo(4,5-d)pyrimidine that exhibits same functional characteristics of the identified analogues. The derivative may be structurally similar by lacking one or more atoms or by been substituted by one or more chemical group.

The term synergic or synergistic as used herein refers to a combination of at least two antimicrobial compounds to produce a combined antimicrobial effect greater than the sum of their separate antimicrobial effects

Biguanide or $HN(C(NH)NH_2)_2$ as used herein refers to

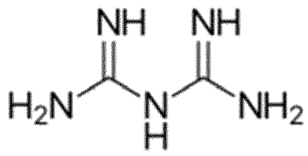

;

Bisbiguanide as used herein refers to chlorhexidine, alexidine, and polyhexylbiguanide;

Chlorhexidine as used herein refers to chlorhexidine base

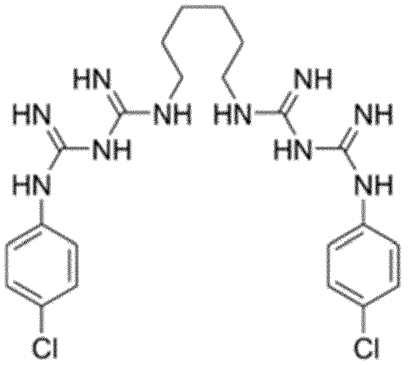

but can also refer to a chlorhexidine salt such as for example chlorhexidine di phosphanilate, chlorhexidine digluconate, chlorhexidine diacetate, chlorhexidine dinitrate, chlorhexidine dihydrochloride, chlorhexidine dichloride, chlorhexidine acetate, chlorhexidine dipropionate, chlorhexidine maleate, chlorhexidine succinate, chlorhexidine thiosulfate, chlorhexidine di-acid phosphate, chlorhexidine malate, chlorhexidine dibenzoate, chlorhexidine diisophtalate, chlorhexidine dilaurate, chlorhexidine distearate, and the like

Alexidine as used herein refers to alexidine base

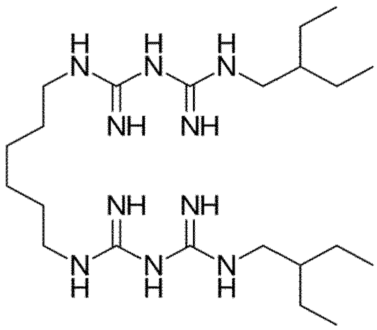

;

but may also refer to alexidine hydrochloride, alexidine dihydrochloride, alexidine monoacetate, alexidine diacetate, alexidine gluconate, alexidine digluconate and mixture thereof.

The antimicrobial composition of the present invention is used against pathogenic microorganisms including bacteria, virus, archaea, protozoa, yeast, or fungi or a mixture thereof, for prevention or treatment of various infection and disease to host mammal (human and animal), particularly on human or animal skin. The Microorganisms as used herein refers to small, but not always microscopic organism. Bacteria may be for example gram positive bacteria such as methicillin-resistant *S. aureus* (MRSA), methicillin-resistant *S. epidermidis* (MRSE), glycopeptide intermediate *S. aureus* (GISA), Coagulase-negative staphylococci (CoNS), Vancomycin-resistant enterococci (VRE), beta-hemolytic *Streptococcus agalactiae* (Group B Streptococcus, GBS); **but also gram negative bacteria** such as *Acinetobacter spp., such as Acinetobacter baumannii, Bordetella pertussis, Campylobacter spp.; Enterobacteriaceae such as Citrobacter spp., Enterobacter spp., Escherichia coli, Klebsiella spp., Salmonella spp., Serratia marcescens, Shigella spp., Yersinia spp.; Haemophilus influenza, Helocobacter pylorilegionella pneumophila, Neisseria spp., Pseudomonas aeruginosa, Vibrio cholera* and the like; **and to yeast or fungi** such as for example *C. albicans, Aspergillus fumigatus, Cryptococcus neoformans, C. tropicalis, C.krusei or a mixture thereof.*

The term skin as used herein refers to a thin layer of tissue forming external covering of a human or animal body or to an ear cavity of human or animal. The term skin also refers to a mucous membrane such as the oral mucosa inside mounth's cavity or such as nasal mucosa inside nose's cavity.

In a preferred embodiment, the antimicrobial composition comprises a synergic or synergistic combination of Triazolo(4,5-d)pyrimidine derivative of formula (I)

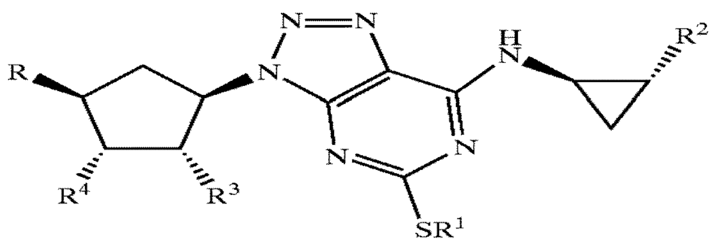

(I)

wherein $R_1$ is $C_{3-5}$ alkyl; $R_2$ is a phenyl group, substituted by one or more halogen atoms; $R_3$ and $R_4$ are both hydroxyl; R is OH, or, $OCH_2CH_2OH$;

or a pharmaceutical acceptable salt, together with **chlorhexidine,** for use in prevention or treatment of infection or inflammation on human or animal, particularly on human or animal skin.

The present antimicrobial composition for use in prevention or treatment of infection or inflammation on human or animal, particularly on human or animal skin has several advantages such as lower cytotoxicity, lower or no allergic reaction, lower risk of antimicrobial resistance since the antimicrobial combination has multiple targets and a quicker killing effect on microorganisms. There is therefore a lower probability that the microorganisms generate mechanisms that protect themselves from antimicrobials effects.

In a most preferred embodiment, the Triazolo(4,5-d)pyrimidine derivatives are the ones including R2 as 4-fluorophenyl or 3,4-difluorophenyl and or R as $OCH_2$ $CH_2OH$.

Most preferred Triazolo(4,5-d)pyrimidine derivatives is (1S,2S,3R,5S)-3-[7-[(1R,25)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol as defined in formula (II) and also called Triafluocyl hereafter;

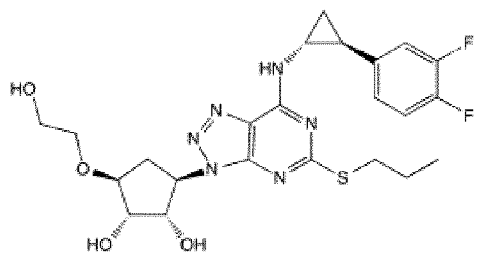

(II)

and a pharmaceutical acceptable salt or solvate thereof, or a solvate of such a salt.

Another most preferred Triazolo(4,5-d)pyrimidine derivative is (1S,2R,3S,4R)-4-[7-[[(1R,2S)-2-(3,4-Difluorophenyl)cyclopropyl]amino]-5-(propylthio)-3H-1,2,3-triazolo[4,5-d]pyrimidin-3-yl]-1,2,3-cyclopentanetriol as defined in formula (III) and also called Fluometacyl hereafter

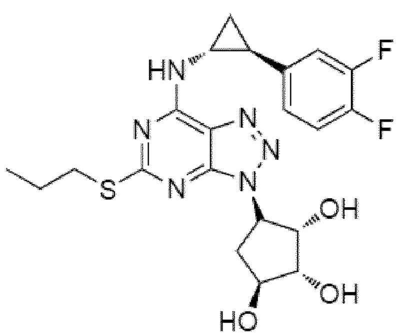

(III)

and a pharmaceutical acceptable salt or solvate thereof, or a solvate of such a salt.

The synergic or synergistic antimicrobial composition of Triazolo(4,5-d)pyrimidine derivatives with chlorhexidine has a broad spectrum of action at low concentration of Triazolo(4,5-d)pyrimidine derivatives and low concentration of

chlorhexidine.

Indeed synergic or synergistic microbial inhibition is reported for triafluocyl at concentration from 40μg/ml to 0.625μg/ml, preferably from 20 μg/ml to 10 μg/ml , most preferably 20μg/ml together with chlorhexidine at concentration of 4μg/ml to 0.25μg/ml, preferably 1 μg/ml to 0.5 μg/ml.

Similarly, synergic or synergistic microbial inhibition is reported for fluometacyl at concentration from 40μg/ml to 0.625 μg/ml, preferably from 20 μg/ml to 5 μg/ml, most preferably from 10 μg/ml to 5 μg/ml together with chlorhexidine at concentration from 4μg/ml to 0.125μg/ml, preferably 4 μg/ml to 0.5 μg/ml.

In a preferred embodiment, the antimicrobial composition for use in prevention or treatment of infection or inflammation on human or animal, particularly on human or animal skin, has a ratio of Triazolo(4,5-d)pyrimidine derivatives to **chlorhexidine** (weight to weight ratio) from 2.5 to 80:1, preferably from 2.5 to 20:1.

In a particular embodiment, the antimicrobial composition for use in prevention or treatment of infection or inflammation on human or animal, particularly on human or animal skin, has a ratio of triafluocyl to chlorhexidine (weight to weight ratio) from 2.5 to 40:1, preferably from 2.5 to 20:1.

In another particular embodiment, the antimicrobial composition for use in prevention or treatment of infection or inflammation on human or animal, particularly on human or animal skin, has a ratio of fluometacyl to chlorhexidine (weight to weight ratio) from 2.5 to 20:1.

The synergic or synergistic antimicrobial composition comprising Triazolo(4,5-d)pyrimidine derivatives and low concentration of chlorhexidine reduces allergic reactions, skin irritation and is efficient for preventing or reducing microbial germs, particularly *Candida albicans, Staphylococcus aureus, Pseudomonas aeruginosa* on surface of objects and human or animal skin.

In a most particular embodiment, the antimicrobial composition, has a ratio of triafluocyl to chlorhexidine (weight to weight ratio) from 2.5:1 to 5:1, for use in prevention or treatment of infection or inflammation caused by *Candida albicans.*

Advantageously, chlorhexidine at a concentration of only 4 μg/ml combined with triafluocyl at a concentration of 10 μg/ml to 20 μg/ml is able to prevent and reduce *Candida albicans.*

In another most particular embodiment, the antimicrobial composition, has a ratio of fluometacyl to chlorhexidine (weight to weight ratio) from 2.5:1 to 5:1, for use in prevention or treatment of infection or inflammation caused by *Candida albicans.*

Advantageously, chlorhexidine at a concentration of only 4 μg/ml combined with fluometacyl at a concentration of 20 μg/ml is also able to prevent and reduce *Candida albicans.*

In another most particular embodiment, the antimicrobial composition, has a ratio of fluometacyl to chlorhexidine (weight to weight ratio) from 5:1 to 20:1, for use in prevention or treatment of infection or inflammation caused *Staphylococcus aureus(MRSA).*

Advantageously, chlorhexidine at a concentration of only 0.5 to 1 μg/ml combined with fluometacyl at a concentration of 5 to 10 μg/ml is also able to prevent and reduce *Staphylococcus aureus(MRSA)* while table 1 above discloses a range of chlorexhidrine between 1 to 8 μg/ml as the Minimal Inhibitory Concentration (MIC) against *Staphylococcus aureus(MRSA)*

In still another most particular embodiment, the antimicrobial composition, has a ratio of fluometacyl to chlorhexidine (weight to weight ratio) from 2.5:1 to 5:1, for use in prevention or treatment of infection or inflammation caused *Pseudomonas aeruginosa.*

In another preferred embodiment, the antimicrobial composition comprises a synergic or synergistic combination of Triazolo(4,5-d)pyrimidine derivative of formula (I)

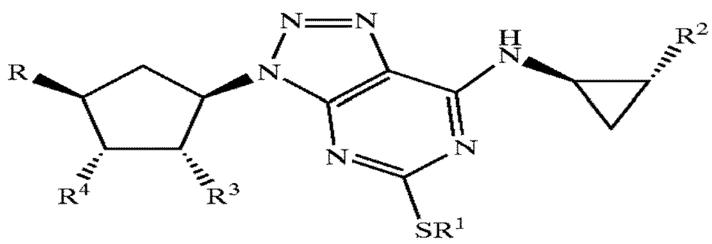

(I)

wherein $R_1$ is C $_{3-5}$ alkyl; $R_2$ is a phenyl group, substituted by one or more halogen atoms; $R_3$ and $R_4$ are both hydroxyl; R is OH, or, $OCH_2CH_2OH$;

or a pharmaceutical acceptable salt, together with **alexidine,** for use in prevention or treatment of infection or inflammation on human or animal, particularly on human or animal skin.

Advantageously, the antimicrobial composition comprising alexidine combined with Triazolo(4,5-d)pyrimidine derivative has a stronger antimicrobial effect than with chlorhexidine with lower allergic reaction; particularly with fluometacyl.

In a most preferred embodiment, the Triazolo(4,5-d)pyrimidine derivatives are the ones including R2 as 4-fluorophenyl or 3,4-difluorophenyl and or R as $OCH_2$ $CH_2OH$.

Most preferred Triazolo(4,5-d)pyrimidine derivatives is (1S,2S,3R,5S)-3-[7-[(1R,25)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol as defined in formula (II) and also called Triafluocyl hereafter;

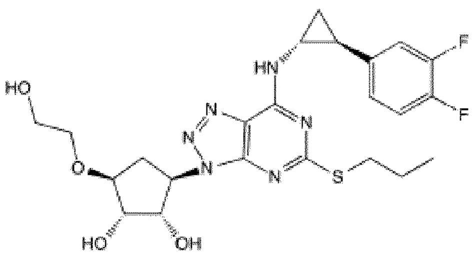

(II)

and a pharmaceutical acceptable salt or solvate thereof, or a solvate of such a salt.

Another most preferred Triazolo(4,5-d)pyrimidine derivative is (1S,2R,3S,4R)-4-[7-[[(1R,2S)-2-(3,4-difluorophenyl)cyclopropyl]amino]-5-(propylthio)-3H-1,2,3-triazolo[4,5-d]pyrimidin-3-yl]-1,2,3-cyclopentanetriol as defined in formula (III) and also called Fluometacyl herein

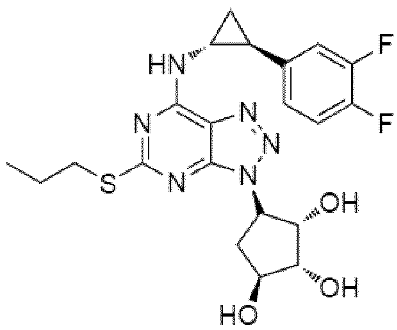

(III)

and a pharmaceutical acceptable salt or solvate thereof, or a solvate of such a salt.

The synergic or synergistic combination of Triazolo(4,5-d)pyrimidine derivative of formula (I) with alexidine is advantageously soluble in aqueous environment or aqueous medium and confers an efficient antimicrobial effect at low level of Triazolo(4,5-d)pyrimidine derivative and low level of alexidine .

Indeed synergic or synergistic microbial inhibition is reported for triafluocyl at concentration from 10μg/ml to 20μg/ml, preferably from 20 μg/ml together with alexidine at concentration of 0.25μg/ml to 1μg/ml, preferably 1 μg/ml.

Similarly, synergic or synergistic microbial inhibition is reported for fluometacyl at concentration of 20μg/ml with alexidine at concentration from 2μg/ml to 0.0625μg/ml, preferably 2 μg/ml.

In a further preferred embodiment, the ratio of Triazolo(4,5-d)pyrimidine derivative of formula (I) with alexidine in the antimicrobial composition is from 5:1 to 320:1 (weight to weight ratio); most preferably from 5:1 to 40:1.

In a particular embodiment, the antimicrobial composition for use in prevention or treatment of infection or inflammation on human or animal, particularly on human or animal skin, has a ratio of triafluocyl to alexidine (weight to weight ratio) from 5:1 to 80:1.

In another particular embodiment, the antimicrobial composition for use in prevention or treatment of infection or inflammation on human or animal, particularly on human or animal skin, has a ratio of fluometacyl to alexidine (weight to weight ratio) from 10:1 to 320:1.

In a most particular embodiment, the antimicrobial composition, has a ratio of triafluocyl to alexidine (weight to weight ratio) of 10:1, for use in prevention or treatment of infection or inflammation caused by *Pseudomonas aeruginosa.*

In another most particular embodiment, the antimicrobial composition, has a ratio of triafluocyl to alexidine (weight to weight ratio) of 20:1, for use in prevention or treatment of infection or inflammation caused by *Candida albicans.*

In a most particular embodiment, the antimicrobial composition, has a ratio of fluometacyl to alexidine (weight to

weight ratio) of 40:1, for use in prevention or treatment of infection or inflammation caused by *Staphylococcus aureus (MRSA).*

The synergic or synergistic antimicrobial composition for prevention or treatment of microorganisms on human or animal, particularly on human or animal skin may be applied on wounds, burns, ulcers, mucosa and/or infection and/or inflammation that may appear on human or animal skin for a lot of reason including surgery, medical device implantation such as cardiovascular device, medical device insertion such as catheter, cannula, valve or needle insertion, including wounds caused by bacteria, fungi, virus, envenoming by a poisonous animal such as snake or arthropode. Wound care is important to prevent infection to microorganisms from bacteria, fungi or viruses.

The synergic or synergistic antimicrobial composition for prevention or treatment of microorganisms on human or animal, particularly on human or animal skin may also reduce inflammation together with infection, such as for example erythema, phlebitis, hyperplasia..

The synergic or synergistic antimicrobial composition for prevention or treatment of infection or inflammation on human or animal, particularly on human or animal skin may be administered either in vitro or in vivo. For example, by topical, parenteral, rectal, nasal, transdermal, buccal, sublingal application or a combination thereof.

The synergic or synergistic antimicrobial composition for prevention or treatment of infection or inflammation by microorganisms on human or animal, particularly on human or animal skin, may be administered either on their own or with an acceptable pharmaceutical carrier.

The synergic or synergistic antimicrobial composition for use in prevention or treatment infection or inflammation of the skin may be administered directly on the skin to clean the wound or impregnated on a dressing, bandage, cotton, rayon, polyester fabric, polyethylene fabric, activated carbon, polyurethane, polyester polyurethane, polycarbonate polyurethane, polydimethylsiloxane polyurethane, polyurethane foam, fibrous cellulose, patches, and the like.

The synergic or synergistic antimicrobial composition may be incorporated in gel, cream, ointment, foam, aqueous or solvent solution, aerosol or other pharmaceutically acceptable carrier that can be applied onto the wound, oral infection or inflammation.

Chlorhexidine or alexidine or the pharmaceutically salt thereof can be used in an amount of 0.01 - 5% according to the total weight of the antimicrobial composition.

Triazolo(4,5-d)pyrimidine derivative or the pharmaceutically salt thereof can be used in an amount of 5-20% according to the total weight of the antimicrobial composition

The synergic or synergistic antimicrobial composition according to the invention may further comprises different pharmaceutical acceptable excipients, such as auxiliary substances, preservatives, solvent and/or viscosity modulating agents, flavorings, sweeteners, buffering agent and the like.

By solvent, one means for example water, saline or any other physiological solution, ethanol, glycerol, oil such as vegetable oil or a mixture thereof. By viscosity modulating agent on means for example carboxymethylcellulose.

By sweetening agent, one means saccharin, aspartame, acesulfame, inulin, isomalt, dextrose, fructose, galactose, maltitol, sorbitol, trehalose, xylitol, mannitol, sucrose, glucose, stevia, alitame and the like.

Suitable auxiliary substances and pharmaceutical compositions are described in Remington's Pharmaceutical Sciences, 16th ed., 1980, Mack Publishing Co., edited by Oslo et al.

**According to a second aspect** of the invention, there is also provided a synergic or synergistic antimicrobial composition comprising a combination of Fluometacyl represented by formula (III) and also named (15,2R,35,4R)-4-[7-[[(1R,2S)-2-(3,4-difluorophenyl)cyclopropyl]amino]-5-(propylthio)-3H-1,2,3-triazolo[4,5-d]pyrimidin-3-yl]-1,2,3-cyclopentanetriol,

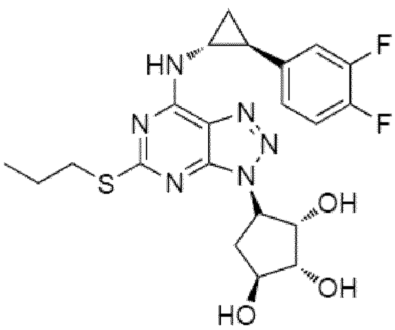

(III)

and a pharmaceutical acceptable salt or solvate thereof, or a solvate of such a salt; together with chlorhexidine or alexidine.

The synergic or synergistic antimicrobial composition of Fluometacyl with chlorhexidine or alexidine have a broad spectrum of action at low concentration of fluometacyl and low concentration of chlorhexidine or alexidine .

The synergic or synergistic antimicrobial composition has several advantages such as lower cytotoxicity, lower or no allergic reaction, lower risk of antimicrobial resistance since the combination has multiple targets and a faster kinetic of killing making microorganisms incapable of counteracting against the antimicrobial composition.

According to a particular embodiment, the antimicrobial composition has a ratio of fluometacyl to chlorhexidine (weight to weight ratio) from 2.5:1 to 20:1.

The synergic or synergistic antimicrobial composition comprising fluometacyl and a low concentration of chlorhexidine reduces allergic reactions or skin irritation and is efficient for preventing or reducing microbial germs, particularly *Candida albicans, Staphylococcus aureus, Pseudomonas aeruginosa* on surface of objects and human or animal skin.

Advantageously, the antimicrobial composition comprising chlorhexidine at a concentration of only 0.5 to 1 μg/ml combined with fluometacyl at a concentration of 5 to 10 μg/ml is also able to prevent and kill *Staphylococcus aureus(MRSA)* while table 1 above discloses a range of chlorhexidrine between 1 to 8 μg/ml as the Minimal Inhibitory Concentration (MIC) against *Staphylococcus aureus(MRSA)*.

According to another particular embodiment, the antimicrobial composition has a ratio of fluometacyl to alexidine (weight to weight ratio) from 10:1 to 320:1.

The antimicrobial composition with Alexidine is effective at a quite lower concentration of alexidine and therefore susceptible to induce less allergic reaction.

Advantageously, synergic or synergistic antimicrobial inhibition is reported for fluometacyl at concentration of 20μg/ml with alexidine at concentration from 0.5μg/ml to 0.0625μg/ml, most preferably 0.5μg/ml.

The antimicrobial composition comprising alexidine at a concentration of 0.5μg/ml to 0.0625μg/ml, combined with fluometacyl at a concentration of 20μg/ml is also able to kill or prevent *Staphylococcus aureus(MRSA)*.

The synergic or synergistic antimicrobial composition may be incorporated in gel, cream, ointment, foam, aqueous or solvent solution, aerosol or other pharmaceutically acceptable carrier.

Chlorhexidine or alexidine or the pharmaceutically salt thereof can be used in an amount of 0.01 - 5% according to the total weight of the antimicrobial composition.

Triazolo(4,5-d)pyrimidine derivative or the pharmaceutically salt thereof can be used in an amount of 5-20% according to the total weight of the antimicrobial composition

The synergic or synergistic antimicrobial composition according to the invention may further comprises different pharmaceutical acceptable excipients, such as auxiliary substances, preservatives, solvent and/or viscosity modulating agents, flavorings, sweeteners, buffering agent and the like.

By solvent, one means for example water, saline or any other physiological solution, ethanol, glycerol, oil such as vegetable oil or a mixture thereof. By viscosity modulating agent on means for example carboxymethylcellulose.

By sweetening agent, one means saccharin, aspartame, acesulfame, inulin, isomalt, dextrose, fructose, galactose, maltitol, sorbitol, trehalose, xylitol, mannitol, sucrose, glucose, stevia, alitame and the like.

Suitable auxiliary substances and pharmaceutical compositions are described in Remington's Pharmaceutical Sciences, 16th ed., 1980, Mack Publishing Co., edited by Oslo et al.

The synergic or synergistic antimicrobial composition may be administered either in vitro or in vivo. For example, by topical, parenteral, rectal, nasal, transdermal, buccal, sublingal application or a combination thereof.

**According to a third aspect,** the invention provides a medical device, biomaterial implants or bioprosthesis comprising the synergic or synergistic antimicrobial composition according to the invention incorporated in a coating or bulk distributed to prevent or treat infection and/or inflammation caused by microorganisms in human or animal, particularly human or animal skin.

Bulk distributed as used herein refers to a uniform or non-uniform distribution of the antimicrobial composition throughout the medical device. The antimicrobial composition can be present at the exterior surface, for example by adherence to the exterior surface of the medical device; but can also optionally be present at points deeper than the exterior surface.

The bulk distribution at points deeper than the exterior surface of medical device is described in US 4,925,668; US 5,165,952, US 5,707,366.

Microorganisms as used herein refers to small, but not always microscopic organism such as bacteria, archaea, protozoa, yeast, fungi. The present invention is directed to pathogenic microorganisms capable to generate disease in human or animal. Bacteria may be for example gram positive bacteria such as methicillin-resistant *S. aureus* (MRSA), methicillin-resistant *S. epidermidis* (MRSE), glycopeptide intermediate *S. aureus* (GISA), Coagulase-negative staphylococci (CoNS), Vancomycin-resistant enterococci (VRE), beta-hemolytic *Streptococcus agalactiae* (Group B Streptococcus, GBS); but also gram negative bacteria such as *Acinetobacter spp., such as Acinetobacter baumannii, Bordetella pertussis, Campylobacter spp.; Enterobacteriaceae such as Citrobacter spp., Enterobacter spp., Escherichia coli, Klebsiella spp., Salmonella spp., Serratia marcescens, Shigella spp., Yersinia spp.; Haemophilus influenza, Helocobacter pylorilegionella pneumophila, Neisseria spp., Pseudomonas aeruginosa, Vibrio cholera* and the like; and to fungi such as for example *C. albicans, Aspergillus fumigatus, Cryptococcus neoformans, C. tropicalis, C.krusei* or a mixture thereof.

The medical device as used herein includes, but is not limited to, any device, tool, instrument, implant, or the like, relating to medicine or the practice of human or veterinary medicine, or intended for use to heal or treat a disease or condition. A medical device may include all natural and synthetic materials and both fibrous and non-fibrous materials. For example, the materials may be comprised of a metal, plastic, glass, ceramic, textile, rubber, polymer, composite material or any other material or combination of materials. Exemplary medical devices include, but are not limited to, any kind of catheter; cannulas; needles; clamps; scalpels; tubes; syringes; spatulas; stents of any size, shape, or placement; coils of any size, shape, or placement; contact lenses; IUDs; peristaltic pump chambers; endotracheal tubes; gastroenteric feeding tubes; arteriovenous shunts; condoms; oxygenator and kidney membranes; gloves; pacemaker leads; wound dressings; metallic pins, plates and screws; metallic artificial hips; artificial knees; and gels; creams and ointments.

The biomaterials, or biomaterial implant as used herein refers to all implantable foreign material for clinical use in human or animal such as for prosthetic joints, pacemakers, implantable cardioverter-defibrillators, catheters such as intravascular or urinary catheters, stent including coronary stent, prosthetic heart valves, bioprostheses, intraocular lens, dental implants, breast implants, endotracheal tubes, gastrostomy tubes and the like.

The bioprothesis, as used herein refers to a prosthesis made of biological material. Examples include heart valves, pericardium, vascular grafts, urinary bladder prostheses, tendon prostheses, hernia patches, surgical mesh and skin substitutes.

In preferred embodiment the medical device, biomaterial implant or bioprosthesis is a catheter or a cardiovascular device.

The medical device, biomaterial implant or bioprosthesis is treated, coated, impregnated or bulk-distributed on at least part of its surface, with the synergic or synergistic antimicrobial composition according to the invention or with the antimicrobial composition for use in prevention or treatment of microbial infection or inflammation on human or animal, particularly on human or animal skin according to the invention.

**According to a fourth aspect,** the invention provides a method of producing the medical device, biomaterial implant or bioprosthesis comprising the synergic or synergistic antimicrobial composition upon at least part of its external or internal surface and comprising the following steps:

i) contacting the surface to be treated with the antimicrobial solution either by dipping, spraying, soaking or wiping
ii) drying the resulting surface obtained at step i)

Alternatively, the method of producing the medical device, biomaterial implant or bioprosthesis is carried out by applying a coating on at least part of the internal or external surface, with the antimicrobial composition; wherein the coating is obtained by the following in-sequence steps:

i) dipping the surface to be coated in a buffer solution of dopamine;

ii) dipping the surface coated with dopamine at step i); in a solution of polymer bearing primary or secondary amine groups; then

iii) dipping the resulting coated surface obtained at step ii) in a mixture of a poly(methacrylamide)-bearing quinone groups of formula (1)

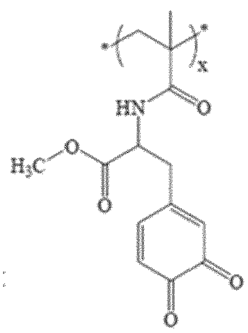

(1)

wherein x is an integer > 1, preferably x is between 1 and 100
with the synergic or synergistic antimicrobial composition;

iv)drying the crosslinked coated surface obtained at step iii) to obtain a coated crosslinked nanogel surface comprising

the antimicrobial composition;

v)optionally repeating steps ii) to iv) to obtain a surface coated with several layers of crosslinked nanogels comprising the synergic or synergistic antimicrobial composition.

More generally and as described In WO2018/122318, the method of producing the medical device, biomaterial implant or bioprosthesis by applying a coating also sometimes called nanoreservoir, comprises applying a first polymer and a second polymer on their surface. The first polymer is bearing one or more catechol moieties and the second polymer comprises a hydrophilic backbone with one or more reactive moieties. The nanoreservoir further comprises the synergic or synergistic antimicrobial composition that is released progressively from within the nanoreservoir.

**According to a fifth aspect,** the invention provides a method of microbial killing or prevention of microbial growth in biofilm formation comprising using, by applying on a surface, an effective amount or concentration of the antimicrobial composition according to the invention or of the antimicrobial composition for use in prevention or treatment of infection or inflammation on human or animal, particularly on human or animal skin.

In a preferred embodiment, the effective amount or concentration is in the range of 0.5-20mg/L of the Triazolo(4,5-d)pyrimidine derivative and 0.1-5mg/L of chlorhexidine.

In another preferred embodiment, the effective amount or concentration is in the range of 0.5-20 mg/L of the Triazolo(4,5-d)pyrimidine derivative and 0.01 to 5 mg/L of alexidine.

By surface one means any type of surface such as rubber or plastic surface as for example surface made of polyethylene, polypropylene, polyurethane, polyvinyl chloride, polyvinylpyrrolidone, polytetrafluoroethylene, silicone or the like, or copolymers but also and preferably metallic surface such as stainless steel, silver, gold, titanium, metallic alloys pyrolitic carbon, and the like. It can also be used on bioabsorbable or biomaterial surface such as biological prosthesis or devices which are made of biological material such as for example porcine or bovine pericardium

By microbial killing one means inhibition of microbial biofilm formation either of bacteria or of yeast or fungi or of any other microorganisms.

By prevention of microbial growth, one means prevention or an inhibition of adherence of microorganism on the surface at the first step of biofilm formation, but also and mainly an inhibition in microorganism grow, multiplication, and formation of microcolonies on the surface at step 2. By inhibition of microbial biofilm, one means inhibition of the matrix at the maturation step 3 and inhibition of microorganism dispersion from the matrix in a colonisation step. By inhibition of microbial biofilm, one also means killing microorganisms at all steps of the biofilm formation.

The method of microbial killing or prevention of microorganism growth on a surface is generally applied to biomaterials or medical devices, preferably on implantable foreign material for clinical use in human or animal such as prosthetic devices, pacemakers, implantable cardioverter-defibrillators, all types of catheters, coronary stent, heart valves, intraocular lens and the like but could be extended to other medical devices requesting no microbial contamination such as for example wound dressings, soft tissue fillers containing local anaesthetics, root canal fillers with ancillary medicinal substances and the like.

The method of microbial killing or prevention of microbial growth could also be applied to surface of experimental device in need of such antimicrobial treatment.

The method of microbial killing or prevention of microbial growth on a surface or part of it comprises contacting the surface to be treated with the antimicrobial composition of the invention or the composition for use in prevention or treatment of infection or inflammation on human or animal, particularly on human or animal skin, either by dipping, spraying, soaking or wiping.

Alternatively the method of microbial killing or prevention of microbial growth in biofilm formation comprises , by applying in a polymeric coating on a surface, an effective amount or concentration of the composition according to the invention or the composition for use in prevention or treatment of infection or inflammation on human or animal, particularly on human or animal skin.

The coating may be made of any polymeric support susceptible to incorporate the synergic or synergistic antimicrobial composition such as for example polyurethane, polyester, polycarbonate, polydimethylsiloxane, poly(N-methacryloyl-3,4dihydroxy-L-phenylalenine methyl ester) (also called Pm(DOPA)), polyallylamine, polyvinylamines, polyvinylamides, polyvinylalcohol, poly(meth)acrylates, poly(meth)acrylamide, Polyethylene glycol(PEG) or a polyelectrolyte (cationic, anionic or zwitterionic) or a hydrophilic biopolymer such as a polysaccharide such as chitosan or hyaluronan.

Preferably the coating is made of a first polymer and a second polymer, the first polymer is bearing one or more catechol moieties; and the second polymer comprises a hydrophilic backbone with one or more reactive moieties as described in WO2018/122318.

**According to a sixth aspect,** the invention provides a coating for medical device, biomaterial implants or bioprosthesis comprising the synergic or synergistic antimicrobial composition according to the invention or the composition for use in prevention or treatment of infection or inflammation on human or animal, particularly on human or animal

skin.

The coating for medical device is made of polymer or copolymer susceptible to incorporate the synergic or synergistic antimicrobial composition and may comprise for example polyurethane, polyester, polycarbonate, polydimethylsiloxane, poly(N-methacryloyl-3,4dihydroxy-L-phenylalenine methyl ester) (also called Pm(DOPA)), polyallylamine, polyvinylamines, polyvinylamides, polyvinylalcohol, poly(meth)acrylates, poly(meth)acrylamide, Polyethylene glycol(PEG) or a polyelectrolyte (cationic, anionic or zwitterionic) or a hydrophilic biopolymer such as a polysaccharide such as chitosan or hyaluronan.

**According to a seventh aspect,** the invention provides a wound dressing comprising the synergic or synergistic antimicrobial composition according to the invention or the composition for use in prevention or treatment of infection or inflammation on human or animal, particularly on human or animal skin.

The wound dressing may be any type of dressing used to cover a wound to prevent or treat infection and/or inflammation on human or animal skin. Wound dressing may be in the form of gel (hydrogel), foam, hydrocolloids, gauze, bandage, patch, film and the like.

Wound dressings may be made of woven cotton fabric, elastomer, coated polyurethane, hydrophilic polymer, hydrofibers such as calcium alginate, carboxymethylated cellulose, and the like.

Wound dressing comprising the synergic or synergistic antimicrobial composition are prepared according to techniques well-known in the art such as soaking, wetting, dipping, spraying and the like.

The invention will be further described by means of non-limiting examples only, with references to the following figures and experimental examples.

**Figure 1** shows real-time microcalorimetric measurements of bacterial metabolic activity expressed as the heat flow, providing *P. aeruginosa* as an example of a Gram-negative bacteria strain. The line A stands for control bacteria with vehicle ($mQH_2O$), whereas the lines B, C, D, E, F and G represent bacteria treated with 1, 2, 4, 6, 12 and 24 mg/L of chlorhexidine, respectively.

**Figure 2** shows an effect of the combination of chlorhexidine and triafluocyl on bacterial proliferation for *P. aeruginosa* using microcalorimetric measurements of bacterial metabolic activity. The line A stands for control bacteria with vehicle at 0.66% Ethanol in TSB , whereas the line B represents bacteria treated with 20 mg/L of triafluocyl; C - 1 mg/L of chlorhexidine; D - 1 mg/L of chlorhexidine and 20 mg/L of triafluocyl; E - 2 mg/L of chlorhexidine; F - 4 mg/L of chlorhexidine; G - 6 mg/L of chlorhexidine; H - 12 mg/L of chlorhexidine; I - 4 mg/L of chlorhexidine and 20 mg/L of triafluocyl; J - 6 mg/L of chlorhexidine and 20 mg/L of triafluocyl; and K - 12 mg/L of chlorhexidine and 20 mg/L of triafluocyl.

**Figure 3** shows real-time microcalorimetric measurements of bacterial metabolic activity expressed as the heat flow, providing *S. aureus* as an example of Gram-positive bacteria strain. The line A stands for control bacteria with a vehicle at 0.66% ethanol in TSB, whereas the line B represents bacteria treated respectively with 1 mg/L of triafluocyl; C - 1 mg/L of chlorhexidine; D - 2 mg/L of chlorhexidine; and E - 1 mg/L of chlorhexidine with 1 mg/L of triafluocyl.

**Figure 4** shows real-time microcalorimetric measurements of bacterial metabolic activity expressed as the heat flow, providing *S. aureus* as an example. The line A stands for control bacteria with a vehicle (ethanol), whereas the line B represents bacteria treated respectively with 0.25 μg/mL of chlorhexidine; C - 0.5 μg/mL of chlorhexidine; D - 1 μg/mL of chlorhexidine; E - 0.25 μg/mL of alexidine; F - 0.5 μg/mL of alexidine; and G - 1 μg/mL of alexidine.

**Figure 5** shows real-time microcalorimetric measurements of bacterial metabolic activity expressed as the heat flow, providing *S. aureus* as an example. The line A stands for control bacteria with a vehicle (ethanol), whereas the line B represents bacteria treated respectively with 0.25 μg/mL of alexidine; C - 0.25 μg/mL of alexidine and 0.25 μg/mL of fluometacyl; D - 0.5 μg/mL of alexidine; and E - 0.5 μg/mL of alexidine with 0.5 μg/mL of fluometacyl.

**Figure 6** shows an effect of the combination of alexidine and fluometacyl on biofilm formation for *P. aeruginosa* using microcalorimetric measurements. The line A stands for control bacteria with a vehicle (ethanol), whereas the lines B and C represent bacteria treated respectively with 4 μg/mL of alexidine and the combination of 4 μg/mL of alexidine with 5 μg/mL of fluometacyl.

**Figure 7** shows real-time microcalorimetric measurements of fungal metabolic activity expressed as the heat flow, providing *C. albicans* as an example. The line A stands for the control growth with a vehicle (ethanol), whereas the line B represents the fungi treated with 1 μg/mL of alexidine; C - 1 μg/mL of alexidine and 5 μg/mL of fluometacyl

**To evaluate synergy between compounds of an antimicrobial composition, a synergy measurement by**

**checkerboard analysis** developed by Emery Pharma, is carried out. It allows to determine the impact on antibiotics combination in comparison to their individual activities. This comparison is then represented as the Fractional Inhibitory Concentration (FIC) index value. The FIC index value takes into account the combination of antibiotics that produces the greatest change from the individual antibiotic's MIC.

To quantify the interactions between the antibiotics being tested (the FIC index), the following equation is used:

$$\frac{A}{MIC_A} + \frac{B}{MIC_B} = FIC_A + FIC_B = \text{FIC Index}$$

wherein A and B are the MIC of respectively A and B in combination (in a single well), and $MIC_A$ and $MIC_B$ are the MIC of A and B individually.

The FIC Index value is then used to categorize the interaction of the two antibiotics tested.

**IF FIC < 0.5, there is synergy;**
**IF FIC > 4, there is antagonism;**
**IF FIC is between 0.5-4, there is additive effect or indifference.**

**Synergy:** when the combination of compounds results in an FIC value of <0.5, then the combination of the compounds increases the inhibitory activity (decrease in MIC) of one or both compounds than the compounds alone.

**Additive or indifference:** when the combination of compounds results in an FIC value of 0.5 - 4, the combination has no increase in inhibitory activity or a slight increase in inhibitory activity from the additive effect of both compounds combined.

**Antagonism:** when the combination of compounds results in an FIC value of >4, the combination of compounds increases the MIC, or lowers the activity of the compounds.

**Example 1: Synergic** or synergistic **effect of triafluocyl and chlorhexidine against gram-negative bacteria** *(Pseudomonas aeruginosa).*

The synergic or synergistic effect of the antimicrobial composition is illustrated hereafter for *Pseudomonas aeruginosa* with a combination triafluocyl/ chlorhexidine as compared with separated triafluocyl or chlorhexidine antimicrobial activities.

Triafluocyl or (15,25,3R,55)-3-[7-[(1R,25)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol is provided by Polpharma whereas chlorhexidine is a chlorhexidine digluconate provided by Merck.

*Pseudomonas aeruginosa (ATCC 15442)* was grown overnight in TSB (tryptic soy broth) medium, before being diluted $1x10^7$ fold in fresh TSB. Subsequently 300 µL aliquots of diluted bacteria suspensions were respectively supplemented with 1, 2, 4, 6, 12 and 24 mg/L of chlorhexidine (digluconate solution, 20% in H2O, Merck). The aqueous solution of chlorhexidine was kept refrigerated according to the manufacturer's technical sheet. Chlorhexidine or $mQH_2O$ as a vehicle were added to the bacterial suspensions to obtain the given concentrations followed by a brief vortexing. Bacterial aliquots were then distributed in the dedicated non-activated inserts of a 48-well plate and grown for 24h or more under static conditions at 37C using Calscreener technology to measure bacterial growth and metabolic activity in Real-Time as described in https://cordis.europa.eu/project/id/784514.

For the study of the combination of chlorhexidine and triafluocyl aforementioned diluted bacterial suspensions were supplemented with either chlorhexidine alone (1, 2, 4, 6, 12 mg/L) or mixed with triafluocyl (20 mg/L, Polpharma). Triafluocyl was stored in absolute ethanol in the refrigerator at a concentration of 3mg/mL. Triafluocyl, chlorhexidine or both, and ethanol as a vehicle were added to the bacterial suspensions to obtain the given concentrations.

Figure 1, illustrates a real-time microcalorimetric measurements of bacterial metabolic activity expressed as the heat flow, providing *P. aeruginosa* as an example. The line A stands for control bacteria with a vehicle ($mQH_2O$), whereas the lines B, C, D, E, F and G represent bacteria treated with 1, 2, 4, 6, 12 and 24 mg/L of chlorhexidine, respectively.

Chlorhexidine fully inhibits bacterial proliferation at the concentration of 24 mg/L.

Figure 2 illustrates an effect of the combination of chlorhexidine and triafluocyl on bacterial proliferation for *P. aeruginosa* using microcalorimetric measurements of bacterial metabolic activity. The line A stands for control bacteria with a vehicle at 0.66% ethanol in TSB, whereas the line B represents bacteria treated with 20 mg/L of triafluocyl; C - 1 mg/L of chlorhexidine; D - 1 mg/L of chlorhexidine and 20 mg/L of triafluocyl; E - 2 mg/L of chlorhexidine; F - 4 mg/L of chlorhexidine; G - 6 mg/L of chlorhexidine; H - 12 mg/L of chlorhexidine; I - 4 mg/L of chlorhexidine and 20 mg/L of triafluocyl; J - 6 mg/L of chlorhexidine and 20 mg/L of triafluocyl; and K - 12 mg/L of chlorhexidine and 20 mg/L of triafluocyl.

Triafluocyl (20 mg/L) does not inhibit the growth of *P. aeruginosa* while the combination of triafluocyl in the

concentration of 20 mg/L with various concentrations of chlorhexidine (1 - 12 mg/L) shows a strong synergic or synergistic effect against the gram-negative bacteria. The full bactericidal effect was reached for 20 mg/L of triafluocyl with 12 mg/L of chlorhexidine.

**Example 2. Synergic effect of triafluocyl and chlorhexidine against gram-positive *bacteria (Staphylococcus aureus (MRSA, ATCC 6538).***

The synergic or synergistic effect of the antimicrobial composition is illustrated hereafter for ***Staphylococcus aureus*** with a combination triafluocyl/ chlorhexidine as compared with separated triafluocyl or chlorhexidine antimicrobial activities.

Triafluocyl or (15,25,3R,55)-3-[7-[(1R,25)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol is provided by Polpharma whereas chlorhexidine is a chlorhexidine digluconate provided by Merck.

***Staphylococcus aureus (MRSA, ATCC 6538)*** was grown overnight in TSB (tryptic soy broth) medium, before being diluted 1x$10^6$ fold in fresh TSB. Subsequently 300 mL aliquots of diluted bacteria suspensions were respectively supplemented with 1 and 2mg/L of chlorhexidine (digluconate solution, 20% in H2O, Merck). To study a synergic or synergistic effect of triafluocyl, 1 mg/L of chlorhexidine was combined with 1 mg/L of triafluocyl (Polpharma). The aqueous solution of chlorhexidine and the ethanol-based solution of triafluocyl were kept refrigerated. Chlorhexidine, triafluocyl or both, and ethanol as a vehicle were added to the bacterial suspensions to obtain the given concentrations followed by a brief vortexing. Bacterial aliquots were then distributed in the dedicated non-activated inserts of a 48-well plate and grown for 24h or more under static conditions at 37C using Calscreener technology to measure bacterial growth and metabolic activity in Real-Time as described in https://cordis.europa.eu/project/id/784514 .

**In** Figure 3, one sees the real-time microcalorimetric measurements of bacterial metabolic activity expressed as the heat flow, providing *S. aureus* as an example of Gram-positive bacteria strain. The line A stands for control bacteria with a vehicle at 0.66% ethanol in TSB, whereas the line B represents bacteria treated respectively with 1 mg/L of triafluocyl; C - 1 mg/L of chlorhexidine; D - 2 mg/L of chlorhexidine; and E - 1 mg/L of chlorhexidine with 1 mg/L of triafluocyl.

Chlorhexidine has a full growth inhibition or bactericidal effect at 2 mg/L, whereas for 1 mg/L a strong shift in a metabolic activity is observed, indicative of reduced bacteria proliferation. Triafluocyl (1 mg/L) doesn't affect the growth of *MRSA*. Whereas the combination of triafluocyl in the concentration of 1 mg/L with the equivalent amount of chlorhexidine shows a strong synergic or synergistic effect against the gram-positive bacteria resulting in the full bacterial growth inhibition.

**Example 3: Alexidine displays stronger antibacterial activity than chlorhexidine.**

*Staphylococcus aureus (MRSA, ATCC 6538)* was grown overnight in TSB (tryptic soy broth) medium, before being diluted 1x$10^6$ fold in fresh TSB. Subsequently 300 μL aliquots of diluted bacteria suspensions were respectively supplemented with 0.25, 0.5 and 1 μg/mL of chlorhexidine (digluconate solution, 20% in H2O, Merck) or alexidine dihydrochloride (stock solution of 3 mg/mL in ethanol, Merck).

The aqueous solution of chlorhexidine and the ethanol-based solution of alexidine were kept refrigerated. Chlorhexidine, alexidine, and ethanol as a vehicle were added to the bacterial suspensions to obtain the given concentrations followed by a brief vortexing. Bacterial aliquots were then distributed in the dedicated non-activated inserts of a 48-well plate and grown for 24h or more under static conditions at 37C using Calscreener technology to measure bacterial growth and metabolic activity in Real-Time as described in https://cordis.europa.eu/project/id/784514.

**In** Figure 4 the real-time microcalorimetric measurements of bacterial metabolic activity is expressed as the heat flow, providing *S. aureus* as an example. The line A stands for control bacteria with a vehicle (ethanol), whereas the line B represents bacteria treated respectively with 0.25 μg/mL of chlorhexidine; C - 0.5 μg/mL of chlorhexidine; D - 1 μg/mL of chlorhexidine; E - 0.25 μg/mL of alexidine; F - 0.5 μg/mL of alexidine; and G - 1 μg/mL of alexidine.

Chlorhexidine at the concentration of 1 μg/mL causes a strong shift in a metabolic activity of *S. aureus.* Whereas alexidine shows noticeably stronger effect seen already at the concentrations of 0.25 and 0.5 μg/mL. In contrast to chlorhexidine, alexidine at the concentration of 1 μg/mL leads to the full growth inhibition.

**Example 4. Synergic** or synergistic **effect of fluometacyl and alexidine against gram-positive *bacteria.***

***Staphylococcus aureus (MRSA, ATCC 6538)*** was grown overnight in TSB (tryptic soy broth) medium, before being diluted 1x$10^6$ fold in fresh TSB. Subsequently 300 μL aliquots of diluted bacteria suspensions were respectively supplemented with 0.25 and 0.5 μg/mL of alexidine dihydrochloride (stock solution of 3 mg/mL in ethanol, Merck). To study a synergic or synergistic effect of fluometacyl, 0.25 μg/mL and 0.5 μg/mL of alexidine was combined with respectively 0.25 μg/mL and 0.5 μg/mL of fluometacyl (ULiege Pharmacy). The ethanol-based solutions of alexidine and fluometacyl were

stored at - 20°C. Alexidine alone or with fluometacyl, and ethanol as a vehicle were added to the bacterial suspensions to obtain the given concentrations followed by a brief vortexing. Bacterial aliquots were then distributed in the dedicated non-activated inserts of a 48-well plate and grown for 24h or more under static conditions at 37C using Calscreener technology to measure bacterial growth and metabolic activity in Real-Time.

**In** Figure 5 real-time microcalorimetric measurements of bacterial metabolic activity is expressed as the heat flow, providing *S. aureus* as an example. The line A stands for control bacteria with a vehicle (ethanol), whereas the line B represents bacteria treated respectively with 0.25 μg/mL of alexidine; C - 0.25 μg/mL of alexidine and 0.25 μg/mL of fluometacyl; D - 0.5 μg/mL of alexidine; and E - 0.5 μg/mL of alexidine with 0.5 μg/mL of fluometacyl.

Combination of fluometacyl with alexidine at the equivalent concentrations shows a noticeable synergic or synergistic effect resulting in the strong growth inhibition of bacteria for the mixture of 0.5 μg/mL of both antimicrobial compounds.

**Example 5. Synergic** or synergistic **effect of fluometacyl and alexidine against gram-negative bacteria.**

***Pseudomonas aeruginosa (ATCC 15442)*** was grown overnight in TSB (tryptic soy broth) medium, before being diluted 1x$10^7$ fold in fresh TSB. Subsequently 300 μL aliquots of diluted bacteria suspensions were respectively supplemented with 4 μg/mL of alexidine dihydrochloride (stock solution of 3 mg/mL in ethanol, Merck). To study a synergic or synergistic effect of fluometacyl, 4 μg/mL of alexidine (the concentration that noticeably shifts the microcalorimetric signal) was combined with 5 μg/mL of fluometacyl (stock solution in ethanol, ULiege Pharmacy). The stock solutions of alexidine and fluometacyl were kept frozen according to the manufacturer's technical sheet. Alexidine alone or with fluometacyl, and ethanol as a vehicle were added to the bacterial suspensions to obtain the given concentrations followed by a brief vortexing. Bacterial aliquots were then distributed in the dedicated non-activated inserts of a 48-well plate and grown for 24h or more under static conditions at 37C using Calscreener technology to measure bacterial growth and metabolic activity in Real-Time as described in https://cordis.europa.eu/project/id/784514.

**In** Figure 6, an effect of the combination of alexidine and fluometacyl on biofilm formation is shown for *P. aeruginosa* using microcalorimetric measurements. The line A stands for control bacteria with a vehicle (ethanol), whereas the lines B and C represent bacteria treated respectively with 4 μg/mL of alexidine and the combination of 4 μg/mL of alexidine with 5 μg/mL of fluometacyl.

Alexidine shows strong growth inhibition of bacteria at the concentration of 4 μg/mL.

Addition of fluometacyl (5 μg/mL) to the mixture with alexidine (4 μg/mL) further potentiates an antibacterial effect underlining a strong synergic or synergistic effect against the gram-negative bacteria.

**Example 6. Synergic** or synergistic **effect of fluometacyl and alexidine against *Candida albicans.***

***Candida albicans* (*3147 ATCC 10231D*)** was grown overnight in MEB (malt extract broth) medium, before being diluted 1x$10^4$ fold in fresh MEB. Subsequently 300 μL aliquots of diluted suspensions of fungi were respectively supplemented with 1 μg/mL of alexidine dihydrochloride (stock solution of 3 mg/mL in ethanol, Merck). To study a synergic or synergistic effect of fluometacyl, 1 μg/mL of alexidine was combined with 5 μg/mL of fluometacyl (ULiege Pharmacy). The ethanol-based solutions of alexidine and fluometacyl were stored at - 20°C. Alexidine alone or with fluometacyl, and ethanol as a vehicle were added to the fungal suspensions to obtain the given concentrations followed by a brief vortexing. The aliquots were then distributed in the dedicated non-activated inserts of a 48-well plate and grown for 24h or more under static conditions at 37°C using Calscreener technology to measure the fungal growth and metabolic activity in Real-Time.

**In** Figure 7, the real-time microcalorimetric measurements of fungal metabolic activity is expressed as the heat flow, providing *C. albicans* as an example. The line A stands for the control growth with a vehicle (ethanol), whereas the line B represents the fungi treated with 1 μg/mL of alexidine; C - 1 μg/mL of alexidine and 5 μg/mL of fluometacyl.

Combination of fluometacyl with alexidine at the given concentrations shows a pronounced synergic or synergistic effect resulting in the strong growth inhibition of *C. albicans*

**Example 7: Synergic** or synergistic **effect of fluometacyl and chlorhexidine against gram-positive *bacteria.* Synergy Checkerboard assay.**

**Protocol. *Staphylococcus aureus (MRSA, ATCC 6538)*** was grown overnight (19h) in TSB (tryptic soy broth) medium. Subsequently the culture was 100x diluted in 4 mL of TSB and grown at 37C under agitation 200 rpm until OD600 reached 0.5. Bacterial culture was then diluted 100x in TSB which corresponds to the range of $5 \times 10^4$ - $5 \times 10^5$ CFU/ml and mixed together with test antimicrobial compounds in 96-well plate according to the following scheme:

- Wells with combination of two antimicrobial compounds: 100 μl of bacteria + 50μl of fluometacyl /TSB + 50μl of

chlorhexidine /TSB

- Wells with one antimicrobial compound only: 100μl of bacteria + 50μl of the antimicrobial compound + 50μl of TSB
- Growth control: 100μl of bacteria + 50μl of TSB + 50μl of TSB
- BLANK: TSB only

Antimicrobial compounds were prepared as follows:
Fluometacyl (4 mg/mL in 100% EtOH) served as a master stock. To obtain the final concentration of 40 μg/ml in the bacterial suspension, initial stock of 160 μg/ml in TSB was prepared and further 2-fold diluted using the following serial dilution scheme:

- (1): 40μg/ml (160μg/ml, prepared in TSB)
- (2): 20μg/ml (80μg/ml, dilution in TSB)
- (3): 10μg/ml (40μg/ml, dilution in TSB)
- (4): 5μg/ml (20μg/ml, dilution in TSB)
- (5): 2.5 μg/ml (10μg/ml, dilution in TSB)
- (6): 1.25μg/ml (5μg/ml, dilution in TSB)

Chlorhexidine acetate (2 mg/mL in H2O) served as a master stock. To obtain the final concentration of 4 μg/ml in the bacterial suspension, initial stock of 16 μg/ml in TSB was prepared and further 2-fold diluted using the following serial dilution scheme:

- (1): 4μg/ml (16 μg/ml, prepared in TSB)
- (2): 2μg/ml (8μg/ml, dilution in TSB)
- (3): 1μg/ml (4μg/ml, dilution in TSB)
- (4): 0,5μg/ml (2μg/ml, dilution in TSB)
- (5): 0,25μg/ml (1μg/ml, dilution in TSB)

Subsequently the antimicrobial compound mixes were co-incubated with MRSA ($5 \times 10^4$ - $5 \times 10^5$ CFU/ml) and growth was measured at the endpoint after incubation at 37°C for 24 hours under shaking at 200 rpm. The difference between OD600 at timepoint 0 h and after 24 hours determines the ΔOD600 and stands for the rate of microbial growth. If the value is close to zero after blank substraction (medium without microbes), that expresses the MIC value for an antimicrobial used. .

Synergy evaluation resulting from the comparison between activity of the combination of the antimicrobial compounds and their individual activities was represented as the Fractional Inhibitory Concentration (FIC) index value. The FIC index value takes into account the combination of molecules that produces the greatest change from the individual drug's minimum inhibitory concentration (MIC). The antimicrobial compound is also called drug herein.

FIC was calculated as followed $\Sigma FICs = FIC_A + FIC_B$, where $FIC_A = MIC_{A+B}/MIC_A$ and $FIC_B = MIC_{B+A}/MIC_B$. Values of $MIC_{A+B}$ are the MIC of combination of antibiotics (mixed in a single well) and $MIC_A$ and $MIC_B$ are the MIC of each drug individually.

FIC values were categorized as follows (according to Emery Pharma described at https://emerypharma.com/solutions/cell-microbiology-services/antimicrobial-synergy-study-checkerboard-testing):

- Synergy < 0.5
- Partial synergy 0.5 - 1.09
- Additive or indifference 1.1 - 4.0
- Antagonism > 4.0

Table 2 shows the FIC values for the combination of fluometacyl with chlorhexidine tested against *S. aureus* as an example. Concentrations of chlorhexidine presented in the table are 0.25 μg /mL, 0.5 μg /mL and 1 μg /mL, whereas for fluometacyl 2.5 μg/mL, 5 μg/mL and 10 μg/mL

Combination of fluometacyl with chlorhexidine shows a noticeable synergic or synergistic effect for the concentrations respectively 10/0.5, 10/1 and 5/1 and a partial synergy for the concentrations 10/0.25. For the other ratios of the antimicrobial compounds there is additive or indifferent effect of the combination.

**Table 2**

| (μg **/mL)** | **Chlorhexidine (1)** | **Chlorhexidine (0.5)** | **Chlorhexidine (0.25)** |
|---|---|---|---|
| **Fluometacyl (10)** | 0,080351928 | 0,080131901 | 0,799010678 |

(continued)

| (μg /mL) | Chlorhexidine (1) | Chlorhexidine (0.5) | Chlorhexidine (0.25) |
|---|---|---|---|
| **Fluometacyl (5)** | 0,24733268 | 2,064675058 | 1,862645194 |
| **Fluometacyl (2.5)** | 1,940371511 | 2,080511106 | 2,080210324 |

**Example 8: Synergic** or synergistic **effect of triafluocyl and chlorhexidine against gram-positive *bacteria.* Synergy Checkerboard assay.**

**Protocol. *Staphylococcus aureus (MRSA, ATCC 6538)*** was grown overnight (19h) in TSB (tryptic soy broth) medium. Subsequently the culture was 100x diluted in 4 mL of TSB and grown at 37C under agitation 200 rpm until OD600 reached 0.5. Bacterial culture was then diluted 100x in TSB which corresponds to the range of $5 \times 10^4$ - $5 \times 10^5$ CFU/ml and mixed together with test antimicrobial compounds in 96-well plate according to the following scheme:

- Wells with combination of two antimicrobial compounds: 100 μl of bacteria + 50μl of triafluocyl /TSB + 50μl of chlorhexidine /TSB
- Wells with one antimicrobial compound only: 100μl of bacteria + 50μl of the antimicrobial compound + 50μl of TSB
- Growth control: 100μl of bacteria + 50μl of TSB + 50μl of TSB
- BLANK: TSB only

Antimicrobial compounds were prepared as follows:
Triafluocyl (ticagrelor) (4 mg/mL in 100% EtOH)) served as a master stock. To obtain the final concentration of 40 μg/ml in the bacterial suspension, initial stock of 160 μg/ml in TSB was prepared and further 2-fold diluted using the following serial dilution scheme:

- (1): 40μg/ml (160μg/ml, prepared in TSB)
- (2): 20μg/ml (80μg/ml, dilution in TSB)
- (3): 10μg/ml (40μg/ml, dilution in TSB)
- (4): 5μg/ml (20μg/ml, dilution in TSB)
- (5): 2.5 μg/ml (10μg/ml, dilution in TSB)
- (6): 1.25μg/ml (5μg/ml, dilution in TSB)

Chlorhexidine acetate (2 mg/mL in H2O) served as a master stock. To obtain the final concentration of 4 μg/ml in the bacterial suspension, initial stock of 16 μg/ml in TSB was prepared and further 2-fold diluted using the following serial dilution scheme:

- (1): 4μg/ml (16 μg/ml, prepared in TSB)
- (2): 2μg/ml (8μg/ml, dilution in TSB)
- (3): 1μg/ml (4μg/ml, dilution in TSB)
- (4): 0,5μg/ml (2μg/ml, dilution in TSB)
- (5): 0,25μg/ml (1μg/ml, dilution in TSB)

Subsequently the antimicrobial compound mixes were co-incubated with MRSA ($5 \times 10^4$ - $5 \times 10^5$ CFU/ml) and growth was measured at the endpoint after incubation at 37°C for 24 hours under shaking at 200 rpm. The difference between OD600 at timepoint 0 h and after 24 hours determines the ΔOD600 and stands for the rate of microbial growth. If the value is close to zero after blank substraction (medium without microbes), that expresses the MIC value for an antimicrobial used.

Synergy evaluation resulting from the comparison between activity of the combination of the antimicrobial compounds and their individual activities was represented as the Fractional Inhibitory Concentration (FIC) index value. The FIC index value takes into account the combination of molecules that produces the greatest change from the individual drug's minimum inhibitory concentration (MIC).

FIC was calculated as followed $\Sigma FICs = FIC_A + FIC_B$, where $FIC_A = MIC_{A+B}/MIC_A$ and $FIC_B = MIC_{B+A}/MIC_B$. Values of $MIC_{A+B}$ are the MIC of combination of antibiotics (mixed in a single well) and $MIC_A$ and $MIC_B$ are the MIC of each drug individually.

FIC values were categorized as follows (according to Emery Pharma described at https://emerypharma.com/solutions/cell-microbiology-services/antimicrobial-synergy-study-checkerboard-testing):

- Synergy < 0.5
- Partial synergy 0.5 - 1.09
- Additive or indifference 1.1 - 4.0
- Antagonism > 4.0

Table 3 shows the FIC values for the combination of triafluocyl with chlorhexidine tested against *S. aureus* as an example. Concentrations of chlorhexidine presented in the table are 0.25 μg/mL, 0.5 μg/mL and 1 μg/mL, whereas for triafluocyl 2.5 μg/mL, 5 μg/mL, 10 μg/mL and 20 μg/mL.

Combination of triafluocyl with chlorhexidine shows a noticeable partial synergic or synergistic effect for the concentrations respectively 10/1, 20/1 and 20/0.5 (giving the ratio of 40/1). For the other ratios of the antimicrobial compounds there is additive or indifferent effect of the combination.

**Table 3**

| (μg/mL) | CHlorhexidine (1) | CHlorhexidine (0.5) | CHlorhexidine (0.25) |
|---|---|---|---|
| **Triafluocyl (20)** | 0,5135897 | 0,53053184 | 2,05493835 |
| **Triafluocyl (10)** | 0,64154387 | 2,01669424 | 2,18175134 |
| **Triafluocyl (5)** | 1,78580309 | 2,18346947 | 2,24085442 |
| **Triafluocyl (2.5)** | 1,86127015 | 2,04898611 | 2,07463706 |

**Example 9. Synergic** or synergistic **effect of fluometacyl and alexidine against gram-positive *bacteria.* Synergy Checkerboard assay.**

**Protocol. *Staphylococcus aureus (MRSA, ATCC 6538)*** was grown overnight (19h) in TSB (tryptic soy broth) medium. Subsequently the culture was 100x diluted in 4 mL of TSB and grown at 37C under agitation 200 rpm until OD600 reached 0.5. Bacterial culture was then diluted 100x in TSB which corresponds to the range of $5 \times 10^4$ - $5 \times 10^5$ CFU/ml and mixed together with test antimicrobial compounds in 96-well plate according to the following scheme:

- Wells with combination of two antimicrobial compounds: 100 μl of bacteria + 50μl of fluometacyl /TSB + 50μl of alexidine /TSB
- Wells with one antimicrobial compound only: 100μl of bacteria + 50μl of the antimicrobial compound + 50μl of TSB
- Growth control: 100μl of bacteria + 50μl of TSB + 50μl of TSB
- BLANK: TSB only

Antimicrobial compounds were prepared as follows:
Fluometacyl (4 mg/mL in 100% EtOH) served as a master stock. To obtain the final concentration of 40 μg/ml in the bacterial suspension, initial stock of 160 μg/ml in TSB was prepared and further 2-fold diluted using the following serial dilution scheme:

- (1): 40μg/ml (160μg/ml, prepared in TSB)
- (2): 20μg/ml (80μg/ml, dilution in TSB)
- (3): 10μg/ml (40μg/ml, dilution in TSB)
- (4): 5μg/ml (20μg/ml, dilution in TSB)
- (5): 2.5 μg/ml (10μg/ml, dilution in TSB)
- (6): 1.25μg/ml (5μg/ml, dilution in TSB)

Alexidine (2 mg/mL in H2O) served as a master stock. To obtain the final concentration of 4 μg/ml in the bacterial suspension, initial stock of 16 μg/ml in TSB was prepared and further 2-fold diluted using the following serial dilution scheme:

- (1): 4μg/ml (16 μg/ml, prepared in TSB)
- (2): 2μg/ml (8μg/ml, dilution in TSB)
- (3): 1μg/ml (4μg/ml, dilution in TSB)
- (4): 0.5μg/ml (2μg/ml, dilution in TSB)
- (5): 0.25μg/ml (1μg/ml, dilution in TSB)
- (6): 0.125μg/ml (0.5μg/ml, dilution in TSB)

- (7): 0.0625μg/ml (0.25μg/ml, dilution in TSB)

Subsequently the antimicrobial compound mixes were co-incubated with MRSA ($5 \times 10^4$ - $5 \times 10^5$ CFU/ml) and growth was measured at the endpoint after incubation at 37°C for 24 hours under shaking at 200 rpm. The difference between OD600 at timepoint 0 h and after 24 hours determines the ΔOD600 and stands for the rate of microbial growth. If the value is close to zero after blank substraction (medium without microbes), that expresses the MIC value for an antimicrobial used.

Synergy evaluation resulting from the comparison between activity of the combination of the antimicrobial compounds and their individual activities was represented as the Fractional Inhibitory Concentration (FIC) index value. The FIC index value takes into account the combination of molecules that produces the greatest change from the individual drug's minimum inhibitory concentration (MIC).

FIC was calculated as followed $\Sigma FICs = FIC_A + FIC_B$, where $FIC_A = MIC_{A+B}/MIC_A$ and $FIC_B = MIC_{B+A}/MIC_B$. Values of $MIC_{A+B}$ are the MIC of combination of antibiotics (mixed in a single well) and $MIC_A$ and $MIC_B$ are the MIC of each drug individually.

FIC values were categorized as follows (according to Emery Pharma in https://emerypharma.com/solutions/cell-microbiology-services/antimicrobial-synergy-study-checkerboard-testing) :

- Synergy < 0.5
- Partial synergy 0.5 - 1.09
- Additive or indifference 1.1 - 4.0
- Antagonism > 4.0

Table 4 shows the FIC values for the combination of fluometacyl with alexidine tested against *S. aureus* as an example. Concentrations of alexidine presented in the table are 0.0625 μg/mL, 0.125 μg/mL, 0.25 μg/mL and 0.5 μg/mL whereas for fluometacyl 2.5 μg/mL, 5 μg/mL, 10 μg/mL and 20 μg/mL

Combination of fluometacyl with alexidine shows a pronounced synergic or synergistic effect for the concentrations respectively 20/0.5 and a partial synergy for the concentrations 10/0.5, 20/0.25, 20/0.125 and 20/0.0625. For the other ratios of the antimicrobial compounds there is additive or indifferent effect of the combination.

**Table 4**

| (μg/mL) | Alexidine (0.5) | Alexidine (0.25) | Alexidine (0.125) | Alexidine (0.0625) |
|---|---|---|---|---|
| **Fluometacyl (20)** | 0,08146122 | 0,87424597 | 0,7471394 | 1,0504069 |
| **Fluometacyl (10)** | 1,01940834 | 2,21069081 | 2,12475134 | 2,21027891 |
| **Fluometacyl (5)** | 1,91942178 | 2,0905259 | 2,12377523 | 2,1175512 |
| **Fluometacyl (2.5)** | 1,8806061 | 1,98973822 | 2,10289719 | 2,08751991 |

**Example 10. Synergic** or synergistic **effect of triafluocyl and alexidine against gram-positive *bacteria.* Synergy Checkerboard assay.**

**Protocol. *Staphylococcus aureus (MRSA, ATCC 6538)*** was grown overnight (19h) in TSB (tryptic soy broth) medium. Subsequently the culture was 100x diluted in 4 mL of TSB and grown at 37C under agitation 200 rpm until OD600 reached 0.5. Bacterial culture was then diluted 100x in TSB which corresponds to the range of $5 \times 10^4$ - $5 \times 10^5$ CFU/ml and mixed together with test antimicrobial compounds in 96-well plate according to the following scheme:

- Wells with combination of two antimicrobial compounds: 100 μl of bacteria + 50μl of triafluocyl /TSB + 50μl of alexidine /TSB
- Wells with one antimicrobial compound only: 100μl of bacteria + 50μl of the antimicrobial compound + 50μl of TSB
- Growth control: 100μl of bacteria + 50μl of TSB + 50μl of TSB
- BLANK: TSB only

Antimicrobial compounds were prepared as follows:
Triafluocyl (4 mg/mL in 100% EtOH) served as a master stock. To obtain the final concentration of 40 μg/ml in the bacterial suspension, initial stock of 160 μg/ml in TSB was prepared and further 2-fold diluted using the following serial dilution scheme:

- (1): 40μg/ml (160μg/ml, prepared in TSB)

- (2): 20μg/ml (80μg/ml, dilution in TSB)
- (3): 10μg/ml (40μg/ml, dilution in TSB)
- (4): 5μg/ml (20μg/ml, dilution in TSB)
- (5): 2.5 μg/ml (10μg/ml, dilution in TSB)

Alexidine (2 mg/mL in H2O) served as a master stock. To obtain the final concentration of 4 μg/ml in the bacterial suspension, initial stock of 16 μg/ml in TSB was prepared and further 2-fold diluted using the following serial dilution scheme:

- (1): 4μg/ml (16 μg/ml, prepared in TSB)
- (2): 2μg/ml (8μg/ml, dilution in TSB)
- (3): 1μg/ml (4μg/ml, dilution in TSB)
- (4): 0,5μg/ml (2μg/ml, dilution in TSB)
- (5): 0,25μg/ml (1μg/ml, dilution in TSB)
- (6): 0,125μg/ml (0.5μg/ml, dilution in TSB)

Subsequently the antimicrobial compound mixes were co-incubated with MRSA ($5 \times 10^4$ - $5 \times 10^5$ CFU/ml) and growth was measured at the endpoint after incubation at 37°C for 24 hours under shaking at 200 rpm. The difference between OD600 at timepoint 0 h and after 24 hours determines the ΔOD600 and stands for the rate of microbial growth. If the value is close to zero after blank substraction (medium without microbes), that expresses the MIC value for an antimicrobial used.

Synergy evaluation resulting from the comparison between activity of the combination of the antimicrobial compounds and their individual activities was represented as the Fractional Inhibitory Concentration (FIC) index value. The FIC index value takes into account the combination of molecules that produces the greatest change from the individual drug's minimum inhibitory concentration (MIC).

FIC was calculated as followed $\Sigma FICs = FIC_A + FIC_B$, where $FIC_A = MIC_{A+B}/MIC_A$ and $FIC_B = MIC_{B+A}/MIC_B$. Values of $MIC_{A+B}$ are the MIC of combination of antibiotics (mixed in a single well) and $MIC_A$ and $MIC_B$ are the MIC of each drug individually.

FIC values were categorized as follows (according to Emery Pharma at https://emerypharma.com/solutions/cell-microbiology-services/antimicrobial-synergy-study-checkerboard-testing):

- Synergy < 0.5
- Partial synergy 0.5 - 1.09
- Additive or indifference 1.1 - 4.0
- Antagonism > 4.0

Table 5 shows the FIC values for the combination of triafluocyl with alexidine tested against *S. aureus* as an example. Concentrations of alexidine presented in the table are 0.125 μg/mL, 0.25 μg/mL, 0.5 μg/mL and 1 μg/mL whereas for triafluocyl 2.5 μg/mL, 5 μg/mL, 10 μg/mL and 20 μg/mL

Combination of triafluocyl with alexidine shows a pronounced partial synergic or synergistic effect for the concentrations respectively 20/1, 20/0.5 and 20/0.25. For the other ratios of the antimicrobial compounds there is additive or indifferent effect of the combination.

**Table 5**

| (μg/mL) | Alexidine (1) | Alexidine (0.5) | Alexidine (0.25) | Alexidine (0.125) |
|---|---|---|---|---|
| **Triafluocyl (20)** | 0,79028709 | 0,35401104 | 0,5656501 | 1,9617831 |
| **Triafluocyl (10)** | 1,32410541 | 1,54763468 | 2,20439 | 2,13088084 |
| **Triafluocyl (5)** | 1,46722277 | 2,13792919 | 2,09138607 | 2,12812676 |
| **Triafluocyl (2.5)** | 1,49405476 | 1,91302276 | 1,95025566 | 2,00802595 |

**Example 11. Synergic** or synergistic **effect of fluometacyl and chlorhexidine acetate against gram-negative bacteria. Synergy Checkerboard assay.**

**Protocol.** ***Pseudomonas aeruginosa (ATCC 15442)*** was grown overnight (19h) in TSB (tryptic soy broth) medium. Subsequently the culture was 100x diluted in 4 mL of TSB and grown at 37C under agitation 200 rpm until OD600

reached 0.5. Bacterial culture was then diluted 100x in TSB which corresponds to the range of $5 \times 10^4$ - $5 \times 10^5$ CFU/ml and mixed together with test antimicrobial compounds in 96-well plate according to the following scheme:

- Wells with combination of two antimicrobial compounds: 100 μl of bacteria + 50μl of fluometacyl /TSB + 50μl of chlorhexidine /TSB
- Wells with one antimicrobial compound only: 100μl of bacteria + 50μl of the antimicrobial compound + 50μl of TSB
- Growth control: 100μl of bacteria + 50μl of TSB + 50μl of TSB
- BLANK: TSB only

Antimicrobial compounds were prepared as follows:
Triafluocyl (4 mg/mL in 100% EtOH) served as a master stock. To obtain the final concentration of 40 μg/ml in the bacterial suspension, initial stock of 160 μg/ml in TSB was prepared and further 2-fold diluted using the following serial dilution scheme:

- (1): 40μg/ml (160μg/ml, prepared in TSB)
- (2): 20μg/ml (80μg/ml, dilution in TSB)
- (3): 10μg/ml (40μg/ml, dilution in TSB)
- (4): 5μg/ml (20μg/ml, dilution in TSB)
- (5): 2.5 μg/ml (10μg/ml, dilution in TSB)

Chlorhexidine (2 mg/mL in H2O) served as a master stock. To obtain the final concentration of 4 μg/ml in the bacterial suspension, initial stock of 16 μg/ml in TSB was prepared and further 2-fold diluted using the following serial dilution scheme:

- (1): 4μg/ml (16 μg/ml, prepared in TSB)
- (2): 2μg/ml (8μg/ml, dilution in TSB)
- (3): 1μg/ml (4μg/ml, dilution in TSB)
- (4): 0.5μg/ml (2μg/ml, dilution in TSB)

Subsequently the antimicrobial compound mixes were co-incubated with *P. aeruginosa* ($5 \times 10^4$ - $5 \times 10^5$ CFU/ml) and growth was measured at the endpoint after incubation at 37°C for 24 hours under shaking at 200 rpm. The difference between OD600 at timepoint 0 h and after 24 hours determines the ΔOD600 and stands for the rate of microbial growth. If the value is close to zero after blank substraction (medium without microbes), that expresses the MIC value for an antimicrobial used.

Synergy evaluation resulting from the comparison between activity of the combination of the antimicrobial compounds and their individual activities was represented as the Fractional Inhibitory Concentration (FIC) index value. The FIC index value takes into account the combination of molecules that produces the greatest change from the individual drug's minimum inhibitory concentration (MIC).

FIC was calculated as followed $\Sigma FICs = FIC_A + FIC_B$, where $FIC_A = MIC_{A+B}/MIC_A$ and $FIC_B = MIC_{B+A}/MIC_B$. Values of $MIC_{A+B}$ are the MIC of combination of antibiotics (mixed in a single well) and $MIC_A$ and $MIC_B$ are the MIC of each drug individually.

FIC values were categorized as follows (according to Emery Pharma at https://emerypharma.com/solutions/cell-microbiology-services/antimicrobial-synergy-study-checkerboard-testing):

- Synergy < 0.5
- Partial synergy 0.5 - 1.09
- Additive or indifference 1.1 - 4.0
- Antagonism > 4.0

Table 6 shows the FIC values for the combination of fluometacyl with chlorhexidine tested against *P. aeruginosa* as an example. Concentrations of chlorhexidine presented in the table are 0.5 μg/mL, 1 μg/mL, 2 μg/mL and 4 μg/mL whereas for fluometacyl 2.5 μg/mL, 5 μg/mL, 10 μg/mL and 20 μg/mL

Combination of fluometacyl with chlorhexidine shows a pronounced partial synergic or synergistic effect for the concentrations respectively 5/2 and 10/2. For the other ratios of the antimicrobial compounds there is additive or indifferent effect of the combination.

Table 6

| (μg/mL) | Chlorhexidine (4) | Chlorhexidine (2) | Chlorhexidine (1) | Chlorhexidine (0.5) |
|---|---|---|---|---|
| **Fluometacyl (20)** | 1,996932587 | 1,386397806 | 1,419467 | 1,474953 |
| **Fluometacyl (10)** | 1,395916563 | 0,665916089 | 2,220718 | 2,100239 |
| **Fluometacyl (5)** | 1,408912994 | 0,682210936 | 1,991427 | 2,069605 |
| **Fluometacyl (2.5)** | 1,455354141 | 1,892413826 | 2,008993 | 1,964136 |

**Example 12. Synergic** or synergistic **effect of triafluocyl and chlorhexidine acetate against gram-negative bacteria. Synergy Checkerboard assay.**

**Protocol.** ***Pseudomonas aeruginosa (ATCC 15442)*** was grown overnight (19h) in TSB (tryptic soy broth) medium. Subsequently the culture was 100x diluted in 4 mL of TSB and grown at 37C under agitation 200 rpm until OD600 reached 0.5. Bacterial culture was then diluted 100x in TSB which corresponds to the range of $5 \times 10^4$ - $5 \times 10^5$ CFU/ml and mixed together with test antimicrobial compounds in 96-well plate according to the following scheme:

- Wells with combination of two antimicrobial compounds: 100 μl of bacteria + 50μl of triafluocyl /TSB + 50μl of chlorhexidine /TSB
- Wells with one antimicrobial compound only: 100μl of bacteria + 50μl of the antimicrobial compound + 50μl of TSB
- Growth control: 100μl of bacteria + 50μl of TSB + 50μl of TSB
- BLANK: TSB only

Antimicrobial compounds were prepared as follows:
Triafluocyl (4 mg/mL in 100% EtOH) served as a master stock. To obtain the final concentration of 40 μg/ml in the bacterial suspension, initial stock of 160 μg/ml in TSB was prepared and further 2-fold diluted using the following serial dilution scheme:

- (1): 40μg/ml (160μg/ml, prepared in TSB)
- (2): 20μg/ml (80μg/ml, dilution in TSB)
- (3): 10μg/ml (40μg/ml, dilution in TSB)
- (4): 5μg/ml (20μg/ml, dilution in TSB)
- (5): 2.5 μg/ml (10μg/ml, dilution in TSB)

Chlorhexidine (2 mg/mL in H2O) served as a master stock. To obtain the final concentration of 4 μg/ml in the bacterial suspension, initial stock of 16 μg/ml in TSB was prepared and further 2-fold diluted using the following serial dilution scheme:

- (1): 4μg/ml (16 μg/ml, prepared in TSB)
- (2): 2μg/ml (8μg/ml, dilution in TSB)
- (3): 1μg/ml (4μg/ml, dilution in TSB)
- (4): 0.5μg/ml (2μg/ml, dilution in TSB)

Subsequently the antimicrobial compound mixes were co-incubated with P. *aeruginosa* ($5 \times 10^4$ - $5 \times 10^5$ CFU/ml) and growth was measured at the endpoint after incubation at 37°C for 24 hours under shaking at 200 rpm. The difference between OD600 at timepoint 0 h and after 24 hours determines the ΔOD600 and stands for the rate of microbial growth. If the value is close to zero after blank substraction (medium without microbes), that expresses the MIC value for an antimicrobial used.

Synergy evaluation resulting from the comparison between activity of the combination of the antimicrobial compounds and their individual activities was represented as the Fractional Inhibitory Concentration (FIC) index value. The FIC index value takes into account the combination of molecules that produces the greatest change from the individual drug's minimum inhibitory concentration (MIC).

FIC was calculated as followed $\Sigma FICs = FIC_A + FIC_B$, where $FIC_A = MIC_{A+B}/MIC_A$ and $FIC_B = MIC_{B+A}/MIC_B$. Values of $MIC_{A+B}$ are the MIC of combination of antibiotics (mixed in a single well) and $MIC_A$ and $MIC_B$ are the MIC of each drug individually.

FIC values were categorized as follows (according to Emery Pharma at https://emerypharma.com/solutions/cell-microbiology-services/antimicrobial-synergy-study-checkerboard-testing):

- Synergy < 0.5
- Partial synergy 0.5 - 1.09
- Additive or indifference 1.1 - 4.0
- Antagonism > 4.0

Table 7 shows the FIC values for the combination of triafluocyl with chlorhexidine tested against *P. aeruginosa* as an example. Concentrations of chlorhexidine presented in the table are 0.5 μg/mL, 1 μg/mL, 2 μg/mL and 4 μg/mL whereas for triafluocyl 2.5 μg/mL, 5 μg/mL, 10 μg/mL and 20 μg/mL

Combination of triafluocyl with chlorhexidine shows a pronounced partial synergic or synergistic effect for the concentrations respectively 10/2, 20/2 and 20/1. For the other ratios of the antimicrobial compounds there is additive or indifferent effect of the combination.

**Table 6**

| (μg/mL) | Chlorhexidine (4) | Chlorhexidine (2) | Chlorhexidine (1) | Chlorhexidine (0.5) |
|---|---|---|---|---|
| **Triafluocyl (20)** | 1,190251128 | 0,513589699 | 0,530532 | 2,054938 |
| **Triafluocyl (10)** | 1,631127801 | 0,641543867 | 2,016694 | 2,181751 |
| **Triafluocyl (5)** | 1,586223539 | 1,785803089 | 2,183469 | 2,240854 |
| **Triafluocyl (2.5)** | 1,410662107 | 1,861270147 | 2,048986 | 2,074637 |

**Example 13. Synergic** or synergistic **effect of fluometacyl and alexidine against gram-negative bacteria. Synergy Checkerboard assay.**

**Protocol. *Pseudomonas aeruginosa (ATCC 15442)*** was grown overnight (19h) in TSB (tryptic soy broth) medium. Subsequently the culture was 100x diluted in 4 mL of TSB and grown at 37C under agitation 200 rpm until OD600 reached 0.5. Bacterial culture was then diluted 100x in TSB which corresponds to the range of $5 \times 10^4$ - $5 \times 10^5$ CFU/ml and mixed together with test antimicrobial compounds in 96-well plate according to the following scheme:

- Wells with combination of two antimicrobial compounds: 100 μl of bacteria + 50μl of fluometacyl /TSB + 50μl of alexidine /TSB
- Wells with one antimicrobial compound only: 100μl of bacteria + 50μl of the antimicrobial compound + 50μl of TSB
- Growth control: 100μl of bacteria + 50μl of TSB + 50μl of TSB
- BLANK: TSB only

Antimicrobial compounds were prepared as follows:
Fluometacyl (4 mg/mL in 100% EtOH) served as a master stock. To obtain the final concentration of 40 μg/ml in the bacterial suspension, initial stock of 160 μg/ml in TSB was prepared and further 2-fold diluted using the following serial dilution scheme:

- (1): 40μg/ml (160μg/ml, prepared in TSB)
- (2): 20μg/ml (80μg/ml, dilution in TSB)
- (3): 10μg/ml (40μg/ml, dilution in TSB)
- (4): 5μg/ml (20μg/ml, dilution in TSB)
- (5): 2.5 μg/ml (10μg/ml, dilution in TSB)

Alexidine (2 mg/mL in H2O) served as a master stock. To obtain the final concentration of 4 μg/ml in the bacterial suspension, initial stock of 16 μg/ml in TSB was prepared and further 2-fold diluted using the following serial dilution scheme:

- (1): 4μg/ml (16 μg/ml, prepared in TSB)
- (2): 2μg/ml (8μg/ml, dilution in TSB)
- (3): 1μg/ml (4μg/ml, dilution in TSB)
- (4): 0.5μg/ml (2μg/ml, dilution in TSB)
- (5): 0.25μg/ml (1μg/ml, dilution in TSB)

Subsequently the antimicrobial compound mixes were co-incubated with P. *aeruginosa* ($5 \times 10^4$ - $5 \times 10^5$

CFU/ml) and growth was measured at the endpoint after incubation at 37°C for 24 hours under shaking at 200 rpm. The difference between OD600 at timepoint 0 h and after 24 hours determines the ΔOD600 and stands for the rate of microbial growth. If the value is close to zero after blank substraction (medium without microbes), that expresses the MIC value for an antimicrobial used.

Synergy evaluation resulting from the comparison between activity of the combination of the antimicrobial compounds and their individual activities was represented as the Fractional Inhibitory Concentration (FIC) index value. The FIC index value takes into account the combination of molecules that produces the greatest change from the individual drug's minimum inhibitory concentration (MIC).

FIC was calculated as followed $\Sigma FICs = FIC_A + FIC_B$, where $FIC_A = MIC_{A+B}/MIC_A$ and $FIC_B = MIC_{B+A}/MIC_B$. Values of $MIC_{A+B}$ are the MIC of combination of antibiotics (mixed in a single well) and $MIC_A$ and $MIC_B$ are the MIC of each drug individually. FIC values were categorized as follows (according to Emery Pharma at https://emerypharma.com/solutions/cell-microbiology-services/antimicrobial-synergy-study-checkerboard-testing):

- Synergy < 0.5
- Partial synergy 0.5 - 1.09
- Additive or indifference 1.1 - 4.0
- Antagonism > 4.0

Table 8 shows the FIC values for the combination of fluometacyl with alexidine tested against *P. aeruginosa* as an example. Concentrations of alexidine presented in the table are 0.25 μg/mL, 0.5 μg/mL, 1 μg/mL and 2 μg/mL whereas for fluometacyl 2.5 μg/mL, 5 μg/mL, 10 μg/mL and 20 μg/mL

Combination of fluometacyl with alexidine shows a pronounced synergic or synergistic effect for the concentration 20/2 and a partial synergy for the concentrations 10/2, 20/1, 20/0.5 and 20/0.25. For the other ratios of the antimicrobial compounds there is additive or indifferent effect of the combination.

**Table 8**

| (μg/mL) | Alexidine (2) | Alexidine (1) | Alexidine (0.5) | Alexidine (0.25) |
|---|---|---|---|---|
| **Fluometacyl (20)** | 0,081461217 | 0,874246 | 0,747139 | 1,050407 |
| **Fluometacyl (10)** | 1,019408343 | 2,210691 | 2,124751 | 2,210279 |
| **Fluometacyl (5)** | 1,919421784 | 2,090526 | 2,123775 | 2,117551 |
| **Fluometacyl (2.5)** | 1,880606104 | 1,989738 | 2,102897 | 2,08752 |

**Example 14. Synergic** or synergistic **effect of triafluocyl and alexidine against gram-negative bacteria. Synergy Checkerboard assay.**

**Protocol.** ***Pseudomonas aeruginosa (ATCC 15442)*** was grown overnight (19h) in TSB (tryptic soy broth) medium. Subsequently the culture was 100x diluted in 4 mL of TSB and grown at 37C under agitation 200 rpm until OD600 reached 0.5. Bacterial culture was then diluted 100x in TSB which corresponds to the range of $5 \times 10^4$ - $5 \times 10^5$ CFU/ml and mixed together with test antimicrobial compounds in 96-well plate according to the following scheme:

- Wells with combination of two antimicrobial compounds: 100 μl of bacteria + 50μl of triafluocyl /TSB + 50μl of alexidine /TSB
- Wells with one antimicrobial compound only: 100μl of bacteria + 50μl of the antimicrobial compound + 50μl of TSB
- Growth control: 100μl of bacteria + 50μl of TSB + 50μl of TSB
- BLANK: TSB only

Antimicrobial compounds were prepared as follows:
Triafluocyl (4 mg/mL in 100% EtOH) served as a master stock. To obtain the final concentration of 40 μg/ml in the bacterial suspension, initial stock of 160 μg/ml in TSB was prepared and further 2-fold diluted using the following serial dilution scheme:

- (1): 40μg/ml (160μg/ml, prepared in TSB)
- (2): 20μg/ml (80μg/ml, dilution in TSB)
- (3): 10μg/ml (40μg/ml, dilution in TSB)
- (4): 5μg/ml (20μg/ml, dilution in TSB)

- (5): 2.5 μg/ml (10μg/ml, dilution in TSB)

Alexidine (2 mg/mL in H2O) served as a master stock. To obtain the final concentration of 4 μg/ml in the bacterial suspension, initial stock of 16 μg/ml in TSB was prepared and further 2-fold diluted using the following serial dilution scheme:

- (1): 4μg/ml (16 μg/ml, prepared in TSB)
- (2): 2μg/ml (8μg/ml, dilution in TSB)
- (3): 1μg/ml (4μg/ml, dilution in TSB)
- (4): 0.5μg/ml (2μg/ml, dilution in TSB)

Subsequently the antimicrobial compound mixes were co-incubated with P. *aeruginosa* ($5 \times 10^4$ - $5 \times 10^5$ CFU/ml) and growth was measured at the endpoint after incubation at 37°C for 24 hours under shaking at 200 rpm. The difference between OD600 at timepoint 0 h and after 24 hours determines the ΔOD600 and stands for the rate of microbial growth. If the value is close to zero after blank substraction (medium without microbes), that expresses the MIC value for an antimicrobial used.

Synergy evaluation resulting from the comparison between activity of the combination of the antimicrobial compounds and their individual activities was represented as the Fractional Inhibitory Concentration (FIC) index value. The FIC index value takes into account the combination of molecules that produces the greatest change from the individual drug's minimum inhibitory concentration (MIC).

FIC was calculated as followed $\Sigma FICs = FIC_A + FIC_B$, where $FIC_A = MIC_{A+B}/MIC_A$ and $FIC_B = MIC_{B+A}/MIC_B$. Values of $MIC_{A+B}$ are the MIC of combination of antibiotics (mixed in a single well) and $MIC_A$ and $MIC_B$ are the MIC of each drug individually.

FIC values were categorized as follows (according to Emery Pharma at https://emerypharma.com/solutions/cell-microbiology-services/antimicrobial-synergy-study-checkerboard-testing):

- Synergy < 0.5
- Partial synergy 0.5 - 1.09
- Additive or indifference 1.1 - 4.0
- Antagonism > 4.0

Table 9 shows the FIC values for the combination of triafluocyl with alexidine tested against *P. aeruginosa* as an example. Concentrations of alexidine presented in the table are 0.5 μg/mL, 1 μg/mL, 2 μg/mL and 4 μg/mL whereas for triafluocyl 2.5 μg/mL, 5 μg/mL, 10 μg/mL and 20 μg/mL

[] Combination of triafluocyl with alexidine shows a pronounced synergic or synergistic effect for the concentration 20/2 and a partial synergy for the concentrations 20/4 and 20/1. For the other ratios of the antimicrobial compounds there is additive or indifferent effect of the combination.

**Table 9**

| (μg/mL) | Alexidine (4) | Alexidine (2) | Alexidine (1) | Alexidine (0.5) |
|---|---|---|---|---|
| **Triafluocyl (20)** | 0,790287095 | 0,35401104 | 0,56565 | 1,961783 |
| **Triafluocyl (10)** | 1,324105407 | 1,547634675 | 2,20439 | 2,130881 |
| **Triafluocyl (5)** | 1,467222765 | 2,137929189 | 2,091386 | 2,128127 |
| **Triafluocyl (2.5)** | 1,49405476 | 1,913022758 | 1,950256 | 2,008026 |

**Example 15. Synergic** or synergistic **effect of fluometacyl and chlorhexidine acetate against fungi. Synergy Checkerboard assay.**

**[] Protocol.** ***Candida albicans 3147 (ATCC 10231D)*** was grown overnight (24h) in MEB (malt extract broth) medium. Subsequently the culture was 50x diluted in 4 mL of MEB and grown at 37C under agitation 200 rpm until OD600 reached 0.5. Fungal culture was then diluted 100x in TSB which corresponds to the range of $5 \times 10^4$ - $5 \times 10^5$ CFU/ml and mixed together with test antimicrobial compounds in 96-well plate according to the following scheme:

- Wells with combination of two antimicrobial compounds: 100 μl of fungi + 50μl of fluometacyl /MEB + 50μl of chlorhexidine /MEB

- Wells with one antimicrobial compound only: 100µl of fungi + 50µl of the antimicrobial compound + 50µl of MEB
- Growth control: 100µl of fungi + 50µl of MEB + 50µl of MEB
- BLANK: MEB only

Antimicrobial compounds were prepared as follows:
Fluometacyl (4 mg/mL in 100% EtOH) served as a master stock. To obtain the final concentration of 40 µg/ml in the fungal suspension, initial stock of 160 µg/ml in MEB was prepared and further 2-fold diluted using the following serial dilution scheme:

- (1): 40µg/ml (160µg/ml, prepared in MEB)
- (2): 20µg/ml (80µg/ml, dilution in MEB)
- (3): 10µg/ml (40µg/ml, dilution in MEB)
- (4): 5µg/ml (20µg/ml, dilution in MEB)
- (5): 2.5 µg/ml (10µg/ml, dilution in MEB)

Chlorhexidine (2 mg/mL in H2O) served as a master stock. To obtain the final concentration of 4 µg/ml in the fungal suspension, initial stock of 16 µg/ml in MEB was prepared and further 2-fold diluted using the following serial dilution scheme:

- (1): 4µg/ml (16 µg/ml, prepared in MEB)
- (2): 2µg/ml (8µg/ml, dilution in MEB)
- (3): 1µg/ml (4µg/ml, dilution in MEB)
- (4): 0.5µg/ml (2µg/ml, dilution in MEB)

Subsequently the antimicrobial compound mixes were co-incubated with C. *albicans* ($5 \times 10^4$ - $5 \times 10^5$ CFU/ml) and growth was measured at the endpoint after incubation at 37°C for 24 hours under shaking at 200 rpm. The difference between OD600 at timepoint 0 h and after 24 hours determines the ΔOD600 and stands for the rate of microbial growth. If the value is close to zero after blank substraction (medium without microbes), that expresses the MIC value for an antimicrobial used.

Synergy evaluation resulting from the comparison between activity of the combination of the antimicrobial compounds and their individual activities was represented as the Fractional Inhibitory Concentration (FIC) index value. The FIC index value takes into account the combination of molecules that produces the greatest change from the individual drug's minimum inhibitory concentration (MIC).

FIC was calculated as followed $\Sigma$FICs = $FIC_A$ + $FIC_B$, where $FIC_A = MIC_{A+B}/MIC_A$ and $FIC_B = MIC_{B+A}/MIC_B$. Values of $MIC_{A+B}$ are the MIC of combination of antibiotics (mixed in a single well) and $MIC_A$ and $MIC_B$ are the MIC of each drug individually.

FIC values were categorized as follows (according to Emery Pharma at https://emerypharma.com/solutions/cell-microbiology-services/antimicrobial-synergy-study-checkerboard-testing):

- Synergy < 0.5
- Partial synergy 0.5 - 1.09
- Additive or indifference 1.1 - 4.0
- Antagonism > 4.0

Table 10 shows the FIC values for the combination of fluometacyl with chlorhexidine tested against C. *albicans* as an example. Concentrations of chlorhexidine presented in the table are 0.5 µg/mL, 1 µg/mL, 2 µg/mL and 4 µg/mL whereas for fluometacyl 2.5 µg/mL, 5 µg/mL, 10 µg/mL and 20 µg/mL

Combination of fluometacyl with chlorhexidine shows a pronounced synergic or synergistic effect for the concentration 20/4 and a partial synergy for the concentration ratio 10/4. For the other ratios of the antimicrobial compounds there is additive or indifferent effect of the combination.

**Table 10**

| (µg/mL) | Chlorhexidine (4) | Chlorhexidine (2) | Chlorhexidine (1) | Chlorhexidine (0.5) |
|---|---|---|---|---|
| **Fluometacyl (20)** | 0,336498035 | 1,514907249 | 2,040434 | 1,908698 |
| **Fluometacyl (10)** | 0,57573655 | 2,144109315 | 2,026749 | 2,118788 |
| **Fluometacyl (5)** | 1,542489367 | 2,187673499 | 2,030176 | 2,093872 |

(continued)

| (μg/mL) | Chlorhexidine (4) | Chlorhexidine (2) | Chlorhexidine (1) | Chlorhexidine (0.5) |
|---|---|---|---|---|
| **Fluometacyl (2.5)** | 1,788155984 | 2,162382719 | 2,017534 | 2,059182 |

**Example 16. Synergic** or synergistic **effect of triafluocyl and chlorhexidine acetate against fungi. Synergy Checkerboard assay.**

**[] Protocol. *Candida albicans 3147 (ATCC 10231D)*** was grown overnight (24h) in MEB (malt extract broth) medium. Subsequently the culture was 50x diluted in 4 mL of MEB and grown at 37C under agitation 200 rpm until OD600 reached 0.5. Fungal culture was then diluted 100x in TSB which corresponds to the range of $5 \times 10^4$ - $5 \times 10^5$ CFU/ml and mixed together with test antimicrobial compounds in 96-well plate according to the following scheme:

- Wells with combination of two antimicrobial compounds: 100 μl of fungi + 50μl of triafluocyl /MEB + 50μl of chlorhexidine /MEB
- Wells with one antimicrobial compound only: 100μl of fungi + 50μl of the antimicrobial compound + 50μl of MEB
- Growth control: 100μl of fungi + 50μl of MEB + 50μl of MEB
- BLANK: MEB only

Antimicrobial compounds were prepared as follows:
Triafluocyl (4 mg/mL in 100% EtOH) served as a master stock. To obtain the final concentration of 40 μg/ml in the fungal suspension, initial stock of 160 μg/ml in MEB was prepared and further 2-fold diluted using the following serial dilution scheme:

- (1): 40μg/ml (160μg/ml, prepared in MEB)
- (2): 20μg/ml (80μg/ml, dilution in MEB)
- (3): 10μg/ml (40μg/ml, dilution in MEB)
- (4): 5μg/ml (20μg/ml, dilution in MEB)
- (5): 2.5 μg/ml (10μg/ml, dilution in MEB)

Chlorhexidine (2 mg/mL in H2O) served as a master stock. To obtain the final concentration of 4 μg/ml in the fungal suspension, initial stock of 16 μg/ml in MEB was prepared and further 2-fold diluted using the following serial dilution scheme:

- (1): 4μg/ml (16 μg/ml, prepared in MEB)
- (2): 2μg/ml (8μg/ml, dilution in MEB)
- (3): 1μg/ml (4μg/ml, dilution in MEB)
- (4): 0.5μg/ml (2μg/ml, dilution in MEB)

Subsequently the antimicrobial compound mixes were co-incubated with C. *albicans* ($5 \times 10^4$ - $5 \times 10^5$ CFU/ml) and growth was measured at the endpoint after incubation at 37°C for 24 hours under shaking at 200 rpm. The difference between OD600 at timepoint 0 h and after 24 hours determines the ΔOD600 and stands for the rate of microbial growth. If the value is close to zero after blank substraction (medium without microbes), that expresses the MIC value for an antimicrobial used.

Synergy evaluation resulting from the comparison between activity of the combination of the antimicrobial compounds and their individual activities was represented as the Fractional Inhibitory Concentration (FIC) index value. The FIC index value takes into account the combination of molecules that produces the greatest change from the individual drug's minimum inhibitory concentration (MIC).

FIC was calculated as followed $\Sigma FICs = FIC_A + FIC_B$, where $FIC_A = MIC_{A+B}/MIC_A$ and $FIC_B = MIC_{B+A}/MIC_B$. Values of $MIC_{A+B}$ are the MIC of combination of antibiotics (mixed in a single well) and $MIC_A$ and $MIC_B$ are the MIC of each drug individually.

FIC values were categorized as follows (according to Emery Pharma at https://emerypharma.com/solutions/cell-microbiology-services/antimicrobial-synergy-study-checkerboard-testing):

- Synergy < 0.5
- Partial synergy 0.5 - 1.09
- Additive or indifference 1.1 - 4.0

- Antagonism > 4.0

Table 11 shows the FIC values for the combination of triafluocyl with chlorhexidine tested against C. *albicans* as an example. Concentrations of chlorhexidine presented in the table are 0.5 μg/mL, 1 μg/mL, 2 μg/mL and 4 μg/mL whereas for triafluocyl 2.5 μg/mL, 5 μg/mL, 10 μg/mL and 20 μg/mL

Combination of triafluocyl with chlorhexidine shows a pronounced synergic or synergistic effect for the concentrations 10/4 and 20/4. For the other ratios of the antimicrobial compounds there is additive or indifferent effect of the combination.

**Table 11**

| (μg/mL) | Chlorhexidine (4) | Chlorhexidine (2) | Chlorhexidine (1) | Chlorhexidine (0.5) |
|---|---|---|---|---|
| **Triafluocyl (20)** | 0,103155902 | 1,661728493 | 1,93146 | 1,802253 |
| **Triafluocyl (10)** | 0,468649908 | 2,152543972 | 2,093418 | 1,977406 |
| **Triafluocyl (5)** | 1,6156879 | 2,260588572 | 2,285993 | 2,126734 |
| **Triafluocyl (2.5)** | 1,789409008 | 2,29982657 | 2,274047 | 2,202058 |

**Example 17. Synergic** or synergistic **effect of fluometacyl and alexidine acetate against fungi. Synergy Checkerboard assay.**

**Protocol.** ***Candida albicans 3147 (ATCC 10231D)*** was grown overnight (24h) in MEB (malt extract broth) medium. Subsequently the culture was 50x diluted in 4 mL of MEB and grown at 37C under agitation 200 rpm until OD600 reached 0.5. Fungal culture was then diluted 100x in TSB which corresponds to the range of $5 \times 10^4$ - $5 \times 10^5$ CFU/ml and mixed together with test antimicrobial compounds in 96-well plate according to the following scheme:

- Wells with combination of two antimicrobial compounds: 100 μl of fungi + 50μl of fluometacyl /MEB + 50μl of alexidine /MEB
- Wells with one antimicrobial compound only: 100μl of fungi + 50μl of the antimicrobial compound + 50μl of MEB
- Growth control: 100μl of fungi + 50μl of MEB + 50μl of MEB
- BLANK: MEB only

Antimicrobial compounds were prepared as follows:
Fluometacyl (4 mg/mL in 100% EtOH) served as a master stock. To obtain the final concentration of 40 μg/ml in the fungal suspension, initial stock of 160 μg/ml in MEB was prepared and further 2-fold diluted using the following serial dilution scheme:

- (1): 40μg/ml (160μg/ml, prepared in MEB)
- (2): 20μg/ml (80μg/ml, dilution in MEB)
- (3): 10μg/ml (40μg/ml, dilution in MEB)
- (4): 5μg/ml (20μg/ml, dilution in MEB)
- (5): 2.5 μg/ml (10μg/ml, dilution in MEB)

Alexidine (2 mg/mL in H2O) served as a master stock. To obtain the final concentration of 4 μg/ml in the fungal suspension, initial stock of 16 μg/ml in MEB was prepared and further 2-fold diluted using the following serial dilution scheme:

- (1): 4μg/ml (16 μg/ml, prepared in MEB)
- (2): 2μg/ml (8μg/ml, dilution in MEB)
- (3): 1μg/ml (4μg/ml, dilution in MEB)
- (4): 0.5μg/ml (2μg/ml, dilution in MEB)
- (5): 0.25μg/ml (1μg/ml, dilution in MEB)

Subsequently the antimicrobial compound mixes were co-incubated with C. *albicans* ($5 \times 10^4$ - $5 \times 10^5$ CFU/ml) and growth was measured at the endpoint after incubation at 37°C for 24 hours under shaking at 200 rpm. The difference between OD600 at timepoint 0 h and after 24 hours determines the ΔOD600 and stands for the rate of microbial growth. If the value is close to zero after blank substraction (medium without microbes), that expresses the MIC value for an

antimicrobial used.

Synergy evaluation resulting from the comparison between activity of the combination of the antimicrobial compounds and their individual activities was represented as the Fractional Inhibitory Concentration (FIC) index value. The FIC index value takes into account the combination of molecules that produces the greatest change from the individual drug's minimum inhibitory concentration (MIC).

FIC was calculated as followed $\Sigma FICs = FIC_A + FIC_B$, where $FIC_A = MIC_{A+B}/MIC_A$ and $FIC_B = MIC_{B+A}/MIC_B$. Values of $MIC_{A+B}$ are the MIC of combination of antibiotics (mixed in a single well) and $MIC_A$ and $MIC_B$ are the MIC of each drug individually.

FIC values were categorized as follows (according to Emery Pharma at https://emerypharma.com/solutions/cell-microbiology-services/antimicrobial-synergy-study-checkerboard-testing):

- Synergy < 0.5
- Partial synergy 0.5 - 1.09
- Additive or indifference 1.1 - 4.0
- Antagonism > 4.0

Table 12 shows the FIC values for the combination of fluometacyl with alexidine tested against C. *albicans* as an example. Concentrations of alexidine presented in the table are 0.25 µg/mL, 0.5 µg/mL and 1 µg/mL whereas for fluometacyl 2.5 µg/mL, 5 µg/mL, 10 µg/mL and 20 µg/mL

Combination of fluometacyl with alexidine shows a partial synergic or synergistic effect for the concentration 20/1. For the other ratios of the antimicrobial compounds there is additive or indifferent effect of the combination.

**Table 12**

| (µg/mL) | Alexidine (1) | Alexidine (0.5) | Alexidine (0.25) |
|---|---|---|---|
| **Fluometacyl (20)** | 0,863881 | 2,085292 | 1,958605 |
| **Fluometacyl (10)** | 1,586059 | 2,046864 | 2,098184 |
| **Fluometacyl (5)** | 1,862661 | 1,919166 | 2,069205 |
| **Fluometacyl (2.5)** | 2,076309 | 2,070088 | 2,014464 |

**Example 18. Synergic** or synergistic **effect of triafluocyl and alexidine acetate against fungi. Synergy Checkerboard assay.**

**Protocol.** ***Candida albicans 3147 (ATCC 10231D)*** was grown overnight (24h) in MEB (malt extract broth) medium. Subsequently the culture was 50x diluted in 4 mL of MEB and grown at 37C under agitation 200 rpm until OD600 reached 0.5. Fungal culture was then diluted 100x in TSB which corresponds to the range of $5 \times 10^4$ - $5 \times 10^5$ CFU/ml and mixed together with test antimicrobial compounds in 96-well plate according to the following scheme:

- Wells with combination of two antimicrobial compounds: 100 µl of fungi + 50µl of triafluocyl /MEB + 50µl of alexidine /MEB
- Wells with one antimicrobial compound only: 100µl of fungi + 50µl of the antimicrobial compound + 50µl of MEB
- Growth control: 100µl of fungi + 50µl of MEB + 50µl of MEB
- BLANK: MEB only

Antimicrobial compounds were prepared as follows:
Triafluocyl (4 mg/mL in 100% EtOH) served as a master stock. To obtain the final concentration of 40 µg/ml in the fungal suspension, initial stock of 160 µg/ml in MEB was prepared and further 2-fold diluted using the following serial dilution scheme:

- (1): 40µg/ml (160µg/ml, prepared in MEB)
- (2): 20µg/ml (80µg/ml, dilution in MEB)
- (3): 10µg/ml (40µg/ml, dilution in MEB)
- (4): 5µg/ml (20µg/ml, dilution in MEB)
- (5): 2.5 µg/ml (10µg/ml, dilution in MEB)

Alexidine (2 mg/mL in H2O) served as a master stock. To obtain the final concentration of 4 µg/ml in the fungal

suspension, initial stock of 16 μg/ml in MEB was prepared and further 2-fold diluted using the following serial dilution scheme:

- (1): 4μg/ml (16 μg/ml, prepared in MEB)
- (2): 2μg/ml (8μg/ml, dilution in MEB)
- (3): 1μg/ml (4μg/ml, dilution in MEB)
- (4): 0.5μg/ml (2μg/ml, dilution in MEB)

Subsequently the antimicrobial compound mixes were co-incubated with C. *albicans* ($5 \times 10^4$ - $5 \times 10^5$ CFU/ml) and growth was measured at the endpoint after incubation at 37°C for 24 hours under shaking at 200 rpm. The difference between OD600 at timepoint 0 h and after 24 hours determines the ΔOD600 and stands for the rate of microbial growth. If the value is close to zero after blank substraction (medium without microbes), that expresses the MIC value for an antimicrobial used.

Synergy evaluation resulting from the comparison between activity of the combination of the antimicrobial compounds and their individual activities was represented as the Fractional Inhibitory Concentration (FIC) index value. The FIC index value takes into account the combination of molecules that produces the greatest change from the individual drug's minimum inhibitory concentration (MIC).

FIC was calculated as followed $\Sigma FICs = FIC_A + FIC_B$, where $FIC_A = MIC_{A+B}/MIC_A$ and $FIC_B = MIC_{B+A}/MIC_B$. Values of $MIC_{A+B}$ are the MIC of combination of antibiotics (mixed in a single well) and $MIC_A$ and $MIC_B$ are the MIC of each drug individually. FIC values were categorized as follows (according to Emery Pharma described at https://emerypharma.com/solutions/cell-microbiology-services/antimicrobial-synergy-study-checkerboard-testing):

- Synergy < 0.5
- Partial synergy 0.5 - 1.09
- Additive or indifference 1.1 - 4.0
- Antagonism > 4.0

Table 13 shows the FIC values for the combination of triafluocyl with alexidine tested against C. *albicans* as an example. Concentrations of alexidine presented in the table are 0.5 μg/mL, 1 μg/mL, 2 μg/mL and 4 μg/mL whereas for triafluocyl 2.5 μg/mL, 5 μg/mL, 10 μg/mL and 20 μg/mL

Combination of triafluocyl with alexidine shows a synergic or synergistic effect for the concentration 20/1 and a partial synergy for the concentrations 20/2, 20/4 and 10/1. For the other ratios of the antimicrobial compounds there is additive or indifferent effect of the combination.

**Table 13**

| (μg/mL) | Alexidine (4) | Alexidine (2) | Alexidine (1) | Alexidine (0.5) |
|---|---|---|---|---|
| **Triafluocyl (20)** | 0,780468274 | 0,685552096 | 0,358241 | 1,899889 |
| **Triafluocyl (10)** | 1,403127895 | 1,428136802 | 0,959391 | 1,998716 |
| **Triafluocyl (5)** | 1,502679479 | 1,515490902 | 1,847405 | 2,109938 |
| **Triafluocyl (2.5)** | 1,583256526 | 1,439775225 | 1,851618 | 2,047035 |

Claims :

1. A synergic antimicrobial composition comprising a combination of Triazolo(4,5-d)pyrimidine derivative of formula (I)

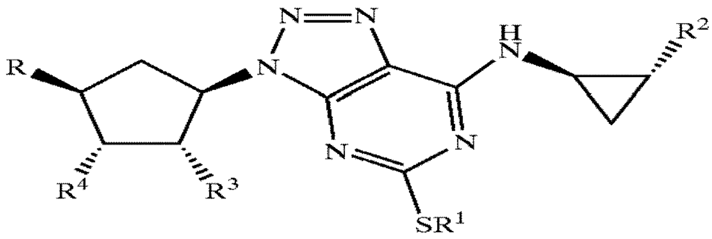

(I)

wherein $R_1$ is $C_{3-5}$ alkyl optionally substituted by one or more halogen atoms; $R_2$ is a phenyl group, optionally substituted by one or more halogen atoms; $R_3$ and $R_4$ are both hydroxyl; R is XOH, wherein X is $CH_2$, $OCH_2CH_2$, or a bond;

or a pharmaceutical acceptable salt or solvate thereof, or a solvate of such a salt provided that when X is $CH_2$ or a bond, $R_1$ is not propyl; when X is $CH_2$ and $R_1$ $CH_2CH_2CF_3$, butyl or pentyl, the phenyl group at $R_2$ must be substituted by fluorine; when X is $OCH_2CH_2$ and $R_1$ is propyl, the phenyl group at $R_2$ must be substituted by fluorine;

together with a bisbiguanide selected from the group consisting of chlorhexidine, alexidine or polyhexylguanidine,

for use in prevention or treatment of infection or inflammation on human or animal, preferably on human or animal skin.

2. The synergic antimicrobial composition for use according to paragraph 1 comprising a combination of Triazolo(4,5-d)pyrimidine derivative of formula (I)

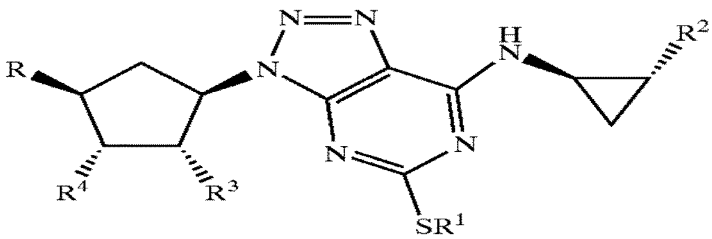

(I)

wherein $R_1$ is $C_{3-5}$ alkyl; $R_2$ is a phenyl group, substituted by one or more halogen atoms; $R_3$ and $R_4$ are both hydroxyl; R is OH, or, $OCH_2CH_2OH$;

or a pharmaceutical acceptable salt,

together with chlorhexidine,

for use in prevention or treatment of infection or inflammation on human or animal, preferably on human or animal skin.

3. The synergic antimicrobial composition for use according to paragraph 1 or 2 characterized-in-that the infection or inflammation is caused by one or more of methicillin-resistant *S. aureus* (MRSA), methicillin-resistant S. *epidermidis* (MRSE), glycopeptide intermediate *S. aureus* (GISA), Coagulase-negative Staphylococci (CoNS), Vancomycin-resistant Enterococci (VRE), beta-hemolytic *Streptococcus agalactiae* (Group B Streptococcus, GBS); *Acinetobacter spp., Acinetobacter baumannii, Bordetella pertussis, Campylobacter spp.; Enterobacteriaceae such as Citrobacter spp., Enterobacter spp., Escherichia coli, Klebsiella spp., Salmonella spp., Serratia marcescens, Shigella spp., Yersinia spp.; Haemophilus influenza, Helocobacter pylorilegionella pneumophila, Neisseria spp., Pseudomonas aeruginosa, Vibrio cholera* and the like; C. *albicans, Aspergillus fumigatus, Cryptococcus neoformans, C. tropicalis, C.krusei* or a mixture thereof.

4. The synergic antimicrobial composition for use according to any one of paragraphs 1 to 3 characterized-in-that the ratio of Triazolo(4,5-d)pyrimidine derivative to chlorhexidine (weight to weight ratio) is from 2.5:1 to 80:1; preferably

from 2.5:1 to 20:1.

5. The synergic antimicrobial composition for use according to any one of paragraphs 1 to 4, wherein the Triazolo(4,5-d)pyrimidine derivative is (15,25,3R,55)-3-[7-[(1R,25)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol also called Triafluocyl.

6. The synergic antimicrobial composition for use according to paragraph 5 characterized-in-that the ratio of triafluocyl to chlorhexidine (weight to weight ratio) is from 2.5:1 to 40:1; preferably from 2.5:1 to 20:1; most preferably from 2.5:1 to 5:1.

7. The synergic antimicrobial composition for use according to paragraph 5 or 6 characterized-in-that triafluocyl concentration is from 40μg/ml to 1.25μg/ml, preferably from 20 μg/ml to 10 μg/ml, most preferably 20μg/ml together with chlorhexidine at concentration of 4μg/ml to 0.25μg/ml, preferably 1 μg/ml to 0.5 μg/ml.

8. The synergic antimicrobial composition for use according to any one of paragraphs 5 to 7 characterized-in-that the ratio triafluocyl to chlorhexidine (weight to weight ratio) is between 2.5:1 to 5:1 for use in prevention or treatment of infection or inflammation caused by *Candida albicans.*

9. The synergic antimicrobial composition for use according to any one of paragraphs 1 to 4, wherein the Triazolo(4,5-d)pyrimidine derivative is (15,2R,35,4R)-4-[7-[[(1R,25)-2-(3,4-difluorophenyl)cyclopropyl]amino]-5-(propylthio)-3H-1,2,3-triazolo[4,5-d]pyrimidin-3-yl]-1,2,3-cyclopentanetriol also called Fluometacyl.

10. The synergic antimicrobial composition for use according to paragraph 9 characterized-in-that fluometacyl concentration is from 40μg/ml to 1.25 μg/ml, preferably from 10 μg/ml to 5 μg/ml together with chlorhexidine concentration from 4μg/ml to 0.0625μg/ml, preferably 4 μg/ml to 0.5 μg/ml.

11. The synergic antimicrobial composition for use according to paragraph 9 or 10 characterized-in-that the ratio fluometacyl to chlorhexidine (weight to weight ratio) is between 2.5:1 and 20:1.

12. The synergic antimicrobial composition for use according to any one of paragraphs 9 to 11 characterized-in-that the ratio fluometacyl to chlorexhidine (weight to weight ratio) is between 2.5:1 and 5:1 for use in prevention or treatment of infection or inflammation caused by *candida albicans.*

13. The synergic antimicrobial composition for use according to any one of paragraph 9 to 11 characterized-in-that the ratio fluometacyl to chlorhexidine (weight to weight ratio) is between 5:1 and 20:1 for use in prevention or treatment of infection or inflammation on human caused by *Staphylococcus aureus (MRSA).*

14. The synergic antimicrobial composition for use according to any one of paragraph 9 to 11 characterized-in-that the ratio fluometacyl to chlorhexidine (weight to weight ratio) is between 2.5:1 and 5:1 for use in prevention or treatment of infection or inflammation on human caused by *Pseudomonas aeruginosa.*

15. The synergic antimicrobial composition for use according to paragraph 1 comprising a combination of Triazolo(4,5-d)pyrimidine derivative of formula (I)

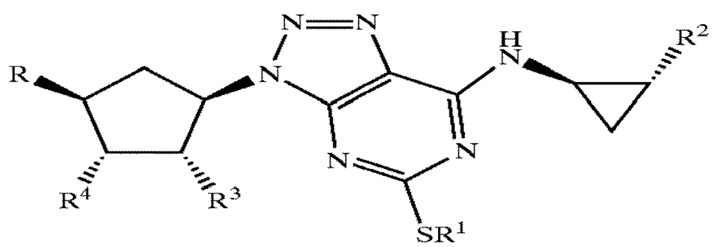

wherein $R_1$ is C $_{3-5}$ alkyl; $R_2$ is a phenyl group, substituted by one or more halogen atoms; $R_3$ and $R_4$ are both hydroxyl; R is OH, or $OCH_2CH_2OH$;

or a pharmaceutical acceptable salt;

together with alexidine for use in prevention or treatment of infection or inflammation on human or animal, preferably on human or animal skin.

16. The synergic antimicrobial composition according to paragraph 15 characterized-in-that the ratio of Triazolo(4,5-d)pyrimidine derivative to alexidine (weight to weight ratio) is from 5:1 to 320:1; preferably from 5:1 to 40:1

17. The synergic antimicrobial composition for use according to any one of paragraphs 15 or 16, wherein the Triazolo(4,5-d)pyrimidine derivative is (15,25,3R,55)-3-[7-[(1R,25)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol also called Triafluocyl.

18. The synergic antimicrobial composition for use according to paragraph 17 characterized-in-that triafluocyl concentration is from 10μg/ml to 20μg/ml, preferably from 20 μg/ml together with alexidine concentration of 0.25μg/ml to 1μg/ml, preferably 1 μg/ml.

19. The synergic antimicrobial composition for use according to any one of paragraphs 17 to 18 characterized-in-that the ratio triafluocyl to alexidine (weight to weight ratio) is between 5:1 to 20:1 for the infection or inflammation caused by *Pseudomonas aeruginosa.*

20. The synergic antimicrobial composition for use according to any one of paragraphs 17 to 18 characterized-in-that the ratio triafluocyl to alexidine (weight to weight ratio) is between 5:1 to 20:1 for the infection or inflammation caused by *Candida albicans.*

21. The synergic antimicrobial composition for use according to any one of paragraphs 15 or 16, wherein the Triazolo(4,5-d)pyrimidine derivative is (15,2R,35,4R)-4-[7-[[(1R,25)-2-(3,4-difluorophenyl)cyclopropyl]amino]-5-(propylthio)-3H-1,2,3-triazolo[4,5-d]pyrimidin-3-yl]-1,2,3-cyclopentanetriol also called Fluometacyl.

22. The synergic antimicrobial composition for use according to paragraph 21 characterized-in-that fluometacyl concentration is 20μg/ml with alexidine at concentration from 2μg/ml to 0.0625μg/ml, preferably 2 μg/ml.

23. The synergic antimicrobial composition for use according to paragraph 21 or 22 characterized-in-that the ratio fluometacyl to alexidine (weight to weight ratio) is between 5:1 to 320:1 for the prevention or treatment of infection or inflammation.

24. The synergic antimicrobial composition for use according to any one of paragraphs 21 to 23 characterized-in-that the ratio fluometacyl to alexidine (weight to weight ratio) is 40:1 for the prevention or treatment of infection or inflammation caused by *Staphylococcus aureus (MRSA).*

25. The synergic antimicrobial composition for use according to any one of paragraphs 21 to 23 characterised-in-that the ratio fluometacyl to alexidine (weight to weight ratio) is 10:1 for the prevention or treatment of infection or inflammation caused by *Pseudomonas aeruginosa.*

26. A medical device, biomaterial implants or bioprosthesis comprising the antimicrobial composition for use as defined in any one of paragraphs 1-7; 9-11 and 15-18; 21-23 incorporated in a coating or bulk distributed.

27. The medical device, biomaterial implants or bioprosthesis according to paragraph 26 which is a catheter or a cardiovascular device.

28. A method of microbial killing or prevention of microbial growth in biofilm formation comprising using, by applying on a surface, an effective amount or concentration of the composition for use a defined in any one of paragraphs 1 to 25 .

29. A method of microbial killing or prevention of microbial growth in biofilm formation comprising, by applying in a polymeric coating on a surface, an effective amount or concentration of the composition for use as defined in paragraphs 1 to 25.

30. The method of microbial killing or prevention of microbial growth according to paragraph 28 or 29 wherein the effective amount or concentration is in the range 0,5-20 mg/L of the Triazolo(4,5-d)pyrimidine derivative and 0.1-5mg mg/L of the chlorhexidine .

31. The method of microbial killing or prevention of microbial growth according to paragraph 28 or 29 wherein the effective amount or concentration is in the range 0,5-20 mg/L of the Triazolo(4,5-d)pyrimidine derivative and 0.01-5mg/L of the alexidine

32. A coating for medical device, biomaterial implants or bioprosthesis comprising the antimicrobial composition for use as defined in paragraphs 1 to 18 and 21 to 23.

33. The coating according to paragraph 32 wherein the medical device, biomaterial implants or bioprosthesis is a catheter or a cardiovascular device.

34. A wound dressing comprising the antimicrobial composition for use as defined in anyone of paragraphs 1 to 18 and 21 to 23.

35. A synergic antimicrobial composition comprising a combination of Triazolo(4,5-d)pyrimidine derivative of formula (I) wherein

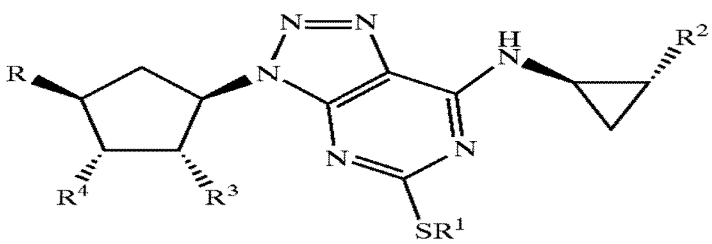

(I)

wherein $R_1$ is C $_{3-5}$ alkyl; $R_2$ is a phenyl group substituted by one or more halogen atoms; $R_3$ and $R_4$ are both hydroxyl; R is OH;
or a pharmaceutical acceptable salt; together with chlorhexidine or alexidine

36. The synergic antimicrobial composition according to paragraph 35 wherein the Triazolo(4,5-d)pyrimidine derivative is (1S,2R,3S,4R)-4-[7-[[(1R,25)-2-(3,4-difluorophenyl)cyclopropyl]amino]-5-(propylthio)-3H-1,2,3-triazolo[4,5-d]pyrimidin-3-yl]-1,2,3-cyclopentanetriol also called Fluometacyl.
37. The synergic antimicrobial composition according to paragraph 36 characterised-in-that fluometacyl concentration is from 40μg/ml to 1.25 μg/ml, preferably from 10 μg/ml to 5 μg/ml together with chlorhexidine concentration from 4μg/ml to 0.125μg/ml, preferably 4 μg/ml to 0.5 μg/ml.
38. The synergic antimicrobial composition according to paragraph 36 or 37 characterized-in-that the ratio fluometacyl to chlorhexidine (weight to weight ratio) is between 2.5:1 and 20:1.
39. The synergic antimicrobial composition according to paragraph 36 characterized-in-that fluometacyl concentration is 20μg/ml with alexidine at a concentration from 2μg/ml to 0.00625μg/ml, preferably 2 μg/ml, most preferably 0.5μg/ml.
40. The synergic antimicrobial composition according to paragraph 36 or 39 characterized-in-that the ratio fluometacyl to alexidine (weight to weight ratio) is between 5:1 to 320:1.
41. A medical device, biomaterial implants or bioprosthesis comprising the antimicrobial composition according to any one of paragraphs 35 to 40 incorporated in a coating or bulk distributed.
42. The medical device, biomaterial implants or bioprosthesis according to paragraph 41 which is a catheter or a cardiovascular device.
43. A method of microbial killing or prevention of microbial growth in biofilm formation comprising using, by applying on a surface, an effective amount or concentration of the composition according to any one of paragraphs 35 to 40.
44. A method of microbial killing or prevention of microbial growth in biofilm formation comprising, by applying in a polymeric coating on a surface, an effective amount or concentration of the composition according to paragraphs 35 to 40.
45. The method of microbial killing or prevention of microbial growth according to paragraph 43 or 44 wherein the effective amount or concentration is in the range 0.5-20 mg/L of the Triazolo(4,5-d)pyrimidine derivative and 0.1-5mg mg/L of the chlorhexidine .
46. The method of microbial killing or prevention of microbial growth according to paragraphs 43 or 44 wherein the effective amount or concentration is in the range 0.5-20 mg/L of the Triazolo(4,5-d)pyrimidine derivative and 0.01-5mg/L of the alexidine
47. A coating for medical device, biomaterial implants or bioprosthesis comprising the antimicrobial composition according to any one of paragraph 35 to 40.
48. The coating according to paragraph 47 wherein the medical device, biomaterial implants or bioprosthesis is a catheter or a cardiovascular device.
49. A wound dressing comprising the antimicrobial composition according to any one of paragraphs 35 to 40.

## Claims

**1.** A synergic antimicrobial composition comprising a combination of Triazolo(4,5-d)pyrimidine derivative of formula (I)

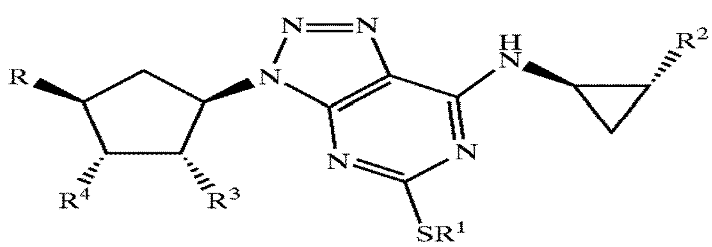

(I)

wherein $R_1$ is $C_{3-5}$alkyl, $R_2$ is a phenyl group, substituted by one or more halogen atoms; $R_3$ and $R_4$ are both hydroxyl; R is XOH, wherein X is $OCH_2CH$ or a bond; or a pharmaceutical acceptable salt;
together with alexidine,
for use in prevention or treatment of infection or inflammation on human or animal, preferably on human or animal skin.

**2.** The synergic antimicrobial composition according to claim 1 characterized-in-that the ratio of Triazolo(4,5-d)pyrimidine derivative to alexidine (weight to weight ratio) is from 5:1 to 320:1; preferably from 5:1 to 40:1.

**3.** The synergic antimicrobial composition for use according to claim 1 or 2 characterized-in-that the Triazolo(4,5-d) pyrimidine derivative is (1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol or also called Triafluocyl and triafluocyl concentration is from 10μg/ml to 20μg/ml, preferably from 20 μg/ml together with alexidine concentration of 0.25μg/ml to 1μg/ml, preferably 1 μg/ml.

**4.** The synergic antimicrobial composition for use according to any one of claim 1 to 3 characterized-in-that the ratio of Triafluocyl to alexidine is between 5:1 to 20:1 for the infection or inflammation caused by *Pseudomonas aeruginosa* or between 5:1 to 20:1 for the infection or inflammation caused by *Candida albicans* or between 80:1 to 20:1 for the prevention or treatment of infection or inflammation caused by *Staphylococcus aureus (MRSA).*

**5.** The synergic antimicrobial composition for use according to claim 1 characterized-in-that the Triazolo(4,5-d) pyrimidine derivative is (1S,2R,3S,4R)-4-[7-[[(1R,2S)-2-(3,4-difluorophenyl)cyclopropyl]amino]-5-(propylthio)-3H-1,2,3-triazolo[4,5-d]pyrimidin-3-yl]-1,2,3-cyclopentanetriol also called Fluometacyl and the ratio to alexidine is between (weight to weight ratio) is 1:1 and between 5:1 and 320:1 for the prevention or treatment of infection or inflammation.

**6.** The synergic antimicrobial composition according to claim 5 characterised-in-that Fluometacyl concentration is 0.5, 5 and 20μg/ml, with alexidine concentration from 2μg/ml to 0.0625μg/ml.

**7.** The synergic antimicrobial composition for use according to claims 5 or 6 characterized-in-that the ratio Fluometacyl to alexidine (weight to weight ratio) is 1:1 and 40:1 for the prevention or treatment of infection or inflammation caused *by Staphylococcus aureus (MRSA);* or 1.25:1 and 10:1 for the prevention or treatment of infection or inflammation caused by *Pseudomonas aeruginosa;* or 5:1 and 20:1 for the prevention or treatment of infection or inflammation caused *by Candida albicans .*

**8.** A medical device, biomaterial implants or bioprosthesis, particularly a catheter or a cardiovascular device, comprising the antimicrobial composition for use as defined in any one of claims 1 incorporated in a coating or bulk distributed.

**9.** A method ex-vivo of microbial killing or prevention of microbial growth in biofilm formation comprising using , by applying on a surface, an effective amount or concentration of the composition for use a defined in any one of claims 1 to 7.

**10.** A method ex-vivo of microbial killing or prevention of microbial growth in biofilm formation comprising, by applying in a polymeric coating on a surface, an effective amount or concentration of the composition for use as defined in any one of claims 1 to 7.

**11.** The method ex-vivo of microbial killing or prevention of microbial growth in biofilm formation according to claim 9 or 10 wherein the effective amount or concentation is in the range 0,5-20 mg/L of Triazolo(4,5-d)pyrimidine derivative and 0.01-5mg/L of the alexidine.

**12.** The synergic antibacterial composition for use according to any one of claim 1 to 7 characterized-in-that the infection or inflammation is caused by one or more of methicillin-resistant S. *aureus* (MRSA), methicillin-resistant S. *epidermidis* (MRSE), glycopeptide intermediate *S. aureus* (GISA), Coagulase-negative Staphylococci (CoNS), Vancomycin-resistant Enterococci (VRE), beta-hemolytic *Streptococcus agalactiae* (Group B Streptococcus, GBS); *Acinetobacter spp., Acinetobacter baumannii, Bordetella pertussis, Campylobacter spp.; Enterobacteriaceae such* as *Citrobacter spp., Enterobacter spp., Escherichia coli, Klebsiella spp., Salmonella spp., Serratia marcescens, Shigella spp., Yersinia spp.; Haemophilus influenza, Helocobacter pylorilegionella pneumophila, Neisseria spp., Pseudomonas aeruginosa, Vibrio cholera* and the like; *C. albicans, Aspergillus fumigatus, Cryptococcus neoformans, C.tropicalis, C.krusei* or a mixture thereof.

**13.** A synergic antibacterial composition comprising a combination of Triazolo(4,5-d)pyrimidine derivative of formula (I)

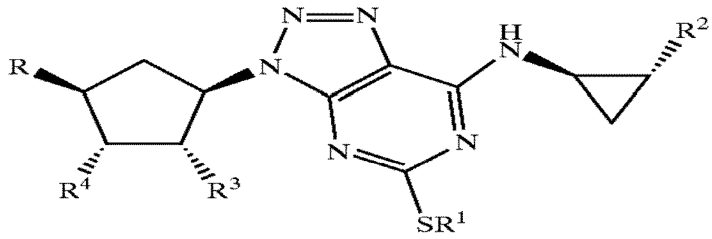

(I)

wherein $R_1$ is $C_{3-5}$ alkyl; $R_2$ is a phenyl group, substituted by one or more halogen atoms; $R_3$ and $R_4$ are both hydroxyl; R is OH; or a pharmaceutically acceptable salt thereof;
together with alexidine.

**14.** The synergic antibacterial composition according to claim 13 wherein the Triazolo(4,5-d)pyrimidine derivative is (1S,2R,3S,4R)-4-[7-[[(1R,2S)-2-(3,4-difluorophenyl)cyclopropyl]amino]-5-(propylthio)-3H-1,2,3-triazolo[4,5-d]pyrimidin-3-yl]-1,2,3-cyclopentanetriol also called Fluometacyl and the ratio Fluometacyl to alexidine (weight to weight ratio) is 1:1 and between 5:1 to 320:1.

**15.** The synergic antimicrobial composition according to claim 14 characterised-in-that Fluometacyl concentration is 0.5 , 5 and 20μg/ml with alexidine at concentration from 2μg/ml to 0.00625μg/ml, preferably 2μg/ml, most preferably 0.5 μg/ml.

**16.** A wound dressing comprising the antimicrobial composition according to any one of claims 13 to 15.

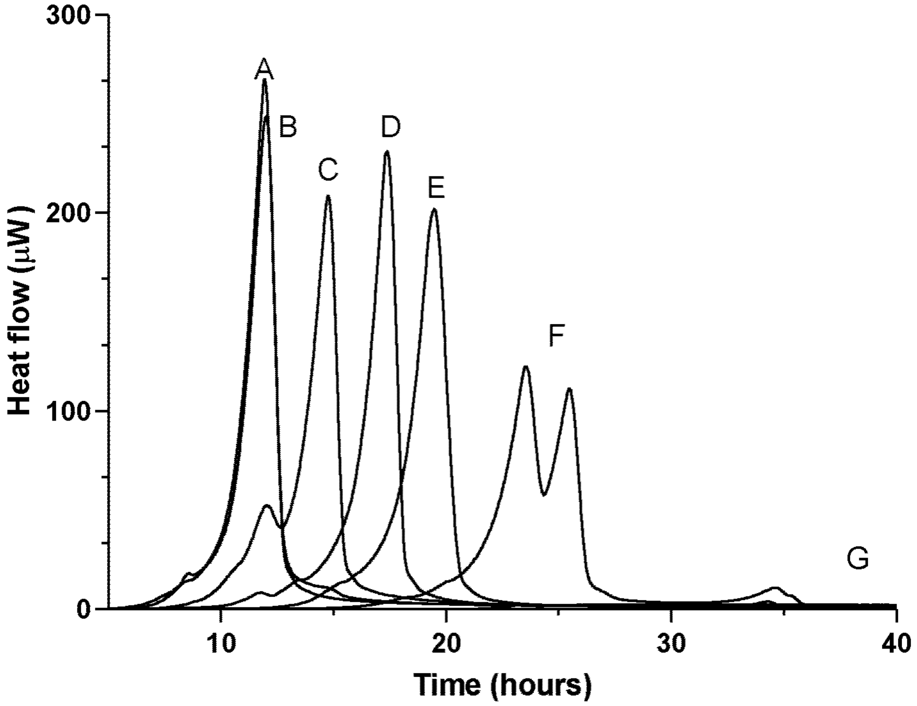
300
200
100
0
Heat flow (μW)
10
20
30
40
Time (hours)
A
B
C
D
E
F
G

FIG.1

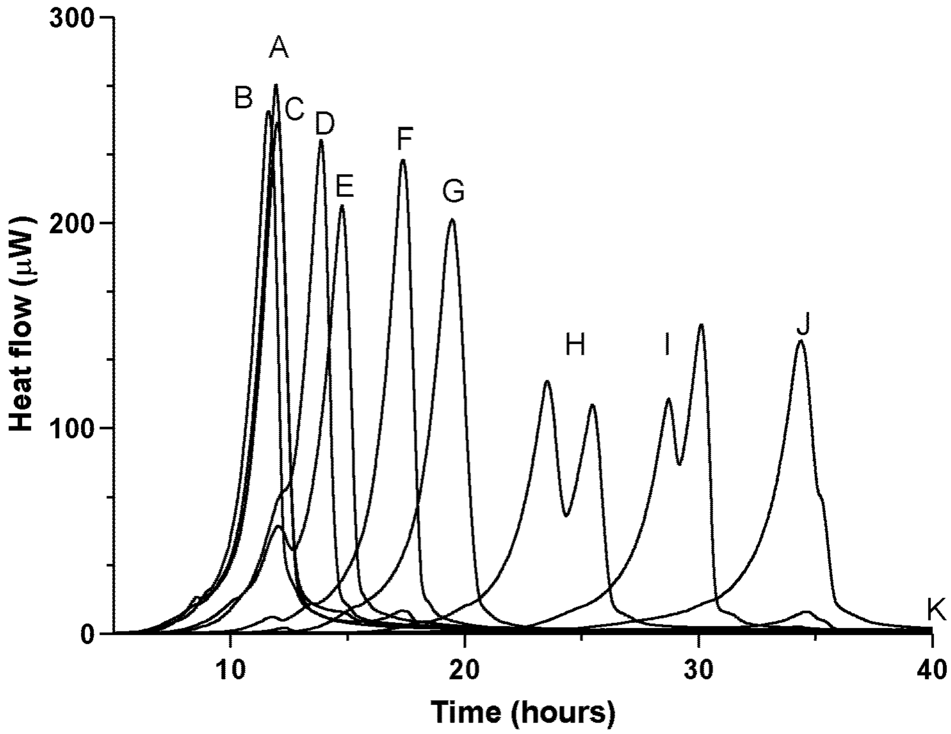
300
200
100
0
Heat flow (μW)
10
20
30
40
Time (hours)
A
B
C
D
E
F
G
H
I
J
K

Fig.2

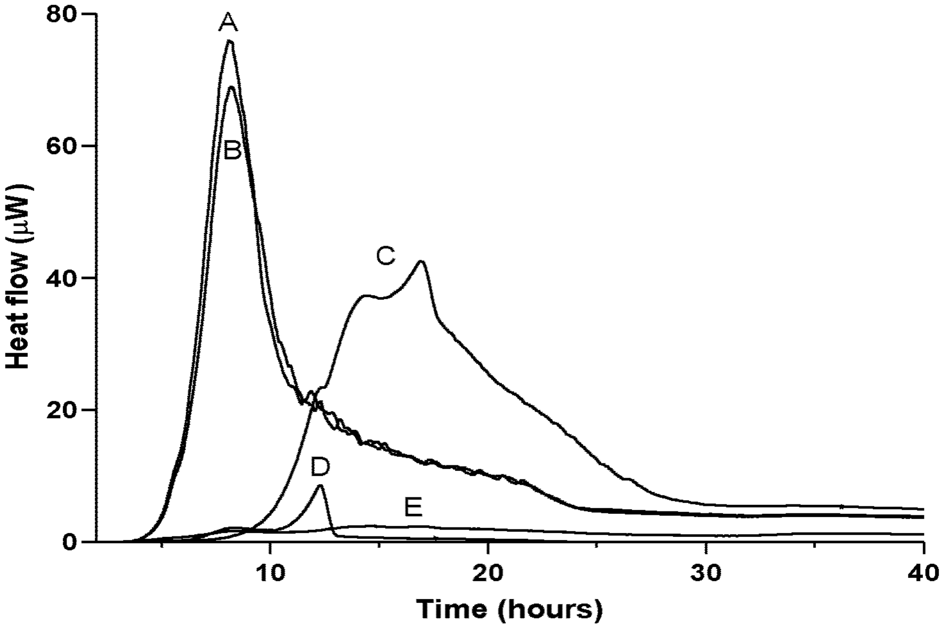
80
60
40
20
0
10
20
30
40
Heat flow (μW)
Time (hours)
A
B
C
D
E

Fig.3

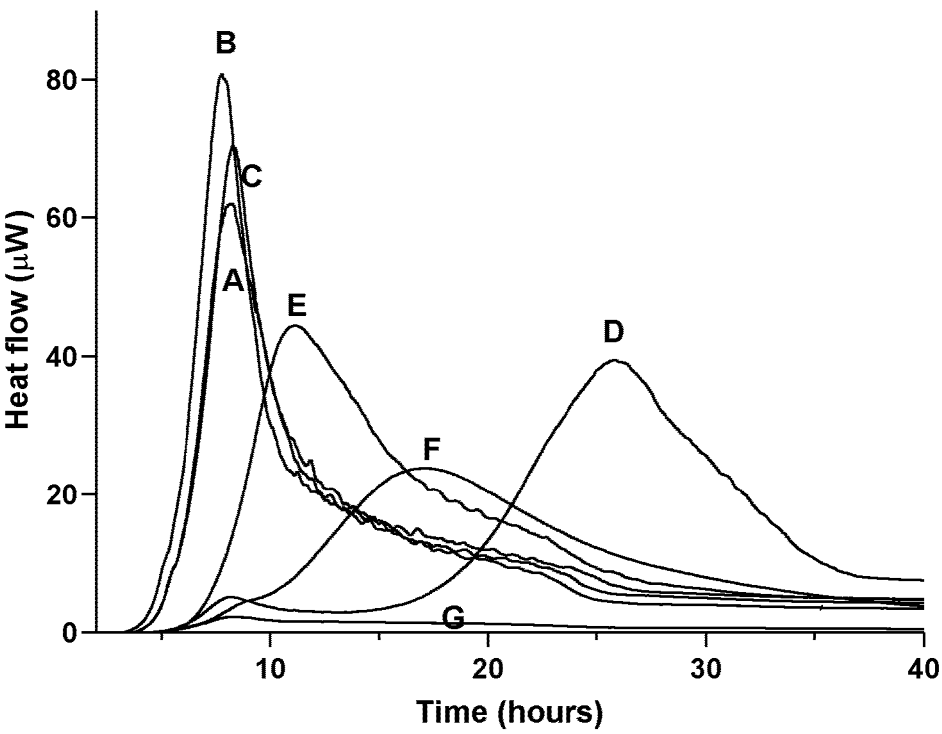
80
60
40
20
0
10
20
30
40
Heat flow (μW)
Time (hours)
B
C
A
E
D
F
G

Fig.4

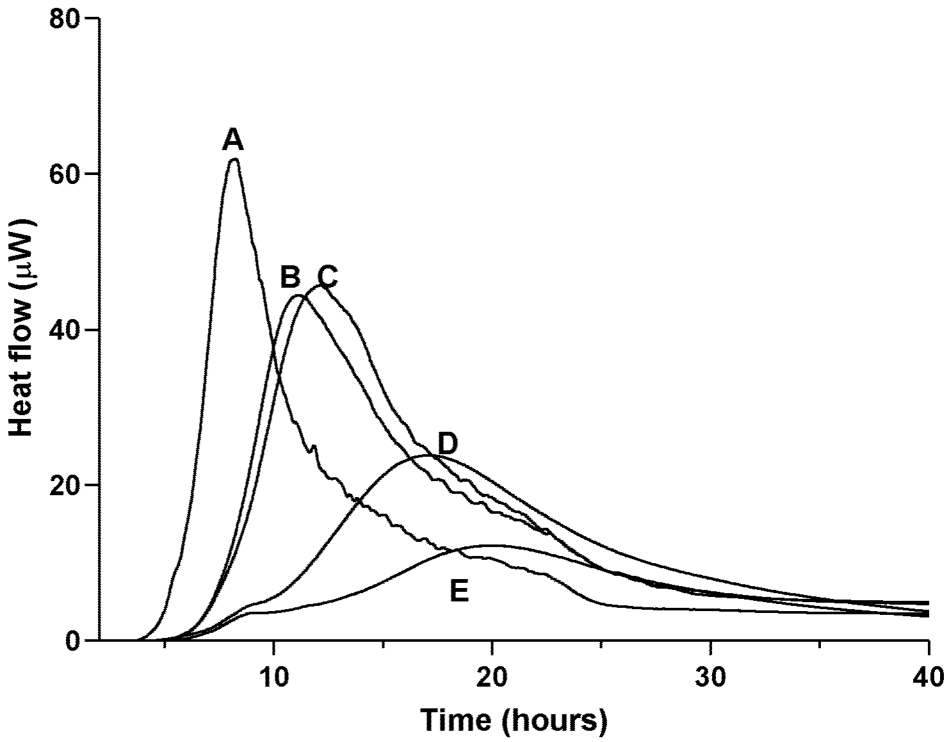

Heat flow (μW)
80
60
40
20
0
A
B
C
D
E
10
20
30
40
Time (hours)


Fig.5

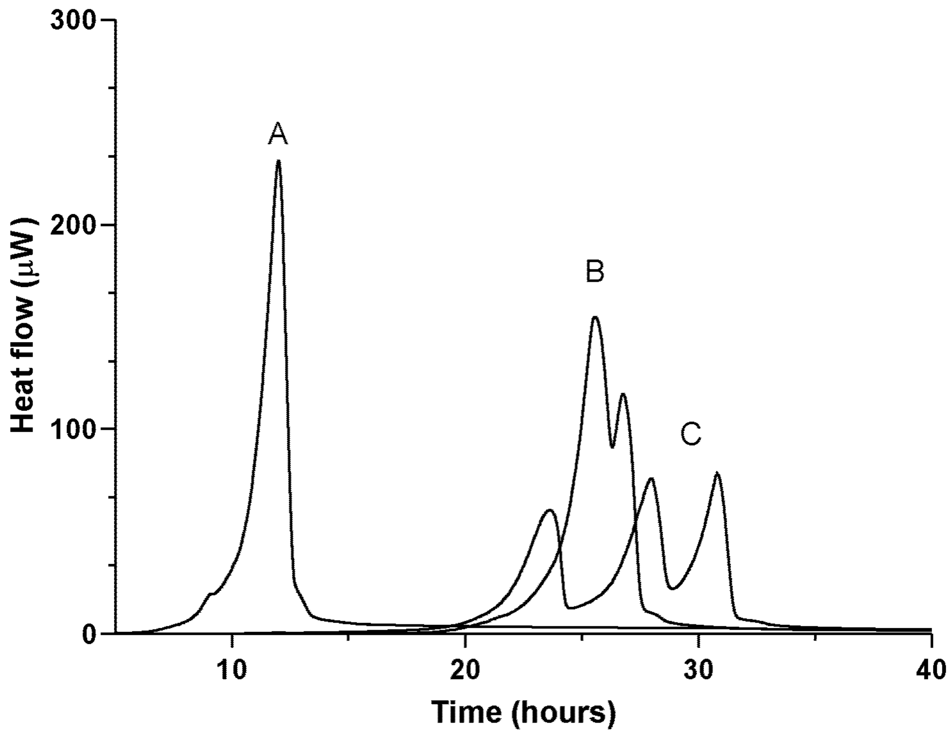

Heat flow (μW)
300
200
100
0
A
B
C
10
20
30
40
Time (hours)


Fig.6

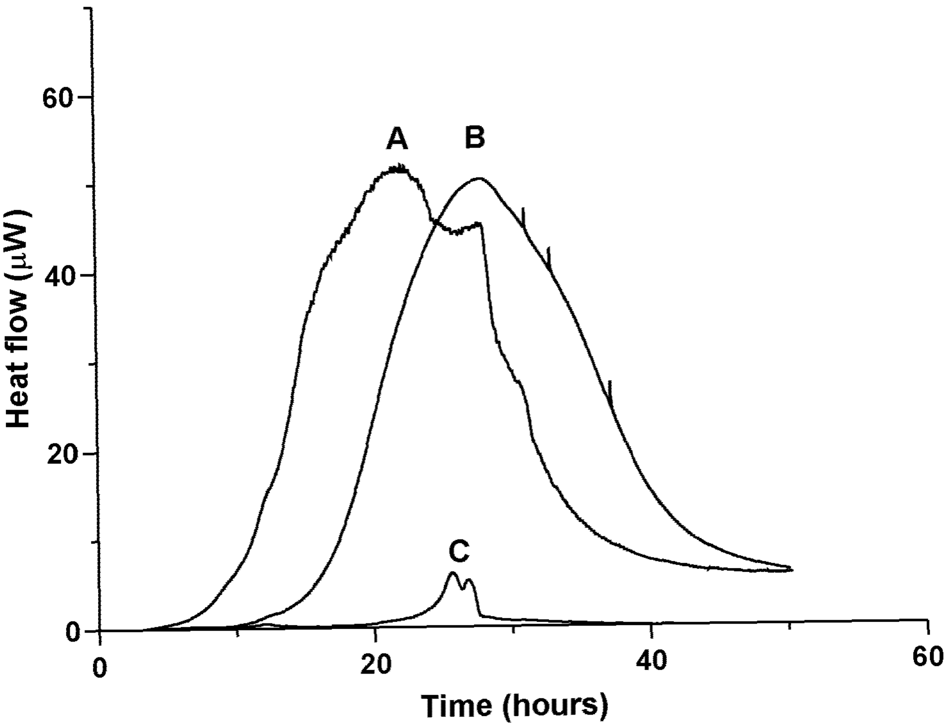


Fig 7.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2968677 B1
- EP 3509598 B1, C. Oury and P.Lancellotti
- US 4925668 A
- US 5165952 A
- US 5707366 A
- WO 2018122318 A


### Non-patent literature cited in the description


- **TM KARPINSKI** ; **AK SZKARADKIEWICZ**. *European Review for Medical and Pharmacological Sciences*, 2015, vol. 19, 1321-1326
- Remington's Pharmaceutical Sciences. Mack Publishing Co, 1980